# EUROPEAN PATENT APPLICATION

(11) **EP 4 198 043 A1**
(43) Date of publication of application: **21.06.2023**
(21) Application number: 21786080.8
(22) Date of filing: 13.08.2021
(51) Int. Cl.: C07K 14/165, C12N 7/04

(54) **METHOD FOR PRODUCING AN ANTIGEN CORRESPONDING TO THE INACTIVATED SARS-COV-2 VIRUS, ANTIGEN CORRESPONDING TO THE INACTIVATED SARS-COV-2 VIRUS, ANTIGENIC COMPOSITION, KITS, AND USES THEREOF**

(30) Priority: 14.08.2020 BR 102020016668; 12.08.2021 BR 102021015972
(71) Applicant: INSTITUTO BUTANTAN, 05503900 São Paulo - SP (BR)
(72) Inventor: OLIVEIRA, Ricardo Das Neves, 05711-000 SÃO PAULO - SP (BR); CHUDZINSKI-TAVASSI, Ana Marisa, 05578-070 São Paulo - SP (BR); TAMBOURGI, Denise Vilarinho, 05595-000 SÃO PAULO - SP (BR); BOTOSSO, Viviane Fongaro, 06036-050 Osasco - SP (BR); JORGE, Soraia Attie Calil, INTERLAGOS SÃO PAULO (BR); ASTRAY, Renato Mancini, 05520-010 SÃO PAULO - SP (BR); MATHOR, Monica Beatriz, 06716-330 SÃO PAULO - SP (BR)
(74) Representative: Plasseraud IP
(86) International application number: PCT/BR2021/050343
(87) International publication number: WO 2022/032368

(57) **Abstract**

The present invention relates to techniques and processes for the production, purification, inactivation and analysis of SARS-CoV-2. The present invention refers to the process to produce an antigen, corresponding to SARS-COV-2 virus, inactivated by gamma irradiation. The process to produce an antigen, corresponding to SARS-COV-2 viruses inactivated by gamma irradiation, is to be used in the production of a new vaccine, antigen for the production of hyperimmune plasma in horses for serum therapy, and in different animal species for the production of antibodies/inputs for research and establishment of serodiagnostic techniques. The present invention refers to the antigenic composition in which it comprises the antigen, corresponding to the inactivated SARS-COV-2 virus, and a pharmaceutically acceptable vehicle, excipient, diluent. It also relates to a Process for producing anti-SARS-CoV-2 immunoglobulin using the SARS-COV-2 virus inactivated by gamma irradiation

## Description

### FIELD OF THE INVENTION:

The present invention relates to techniques and processes for the production, purification, inactivation and analysis of SARS-CoV-2. The present invention refers to the process for producing an antigen, corresponding to SARS-CoV-2 viruses, inactivated by gamma irradiation. The process for producing an antigen, corresponding to SARS-COV-2 viruses inactivated by gamma irradiation, is to be used in the production of a new vaccine, antigen for the production of hyperimmune plasma in horses for serum therapy, and in different animal species for the production of antibodies/inputs for research and establishment of serodiagnostic techniques.

### BACKGROUND OF THE INVENTION

In late 2019, China reported the first case of COVID-19 (Coronavirus Disease 2019), a severe acute respiratory syndrome caused by SARS-CoV-2 (Severe Acute Respiratory Syndrome Coronavirus-2). The new virus has spread rapidly around the world, reaching 167 million cases and 3.5 million deaths in May 2021 (JHUM, 2020). Although vaccines became available in less than a year, approaches to treatment with COVID-19 remain scarce. Many studies have sought different therapeutic interventions, ranging from the use of antiviral and antiparasitic drugs to immunotherapy with interleukins (for example, as interferon-alpha). NK cells or mesenchymal stem cells, as well as plasma transfusion from a convalescent patient (Li *et al.,* 2020). Despite numerous studies, there is still no specific drug to fight COVID-19 and the treatment remains, mostly, symptomatic.

Immunoglobulin of animal origin or plasma from convalescent patients may constitute a specific treatment to ensure the neutralization of the virus in the early stages of infection, especially in patients with risk factors and a high probability of progression to severe disease. This type of approach, also known as passive immunization, originated in the late 19^{th} century, when the first passive immunization tests for diphtheria and tetanus were carried out (Kantha, 1991; Winau & Winau, 2020). Since then, more than 120 years of experience in the production of this type of immunobiological has been accumulated in various parts of the world. Regardless of the origin of the immunoglobulin, the objective of serotherapy is to neutralize the pathogen through antibodies, although phagocytosis and antibody-dependent cellular cytotoxicity can also occur (Van Erp *et al.,* 2019; Gunn *et al.,* 2018), avoiding clinical infection or reducing clinical severity in individuals with recent exposure to a pathogen. Thus, passive antibody therapy is most effective when administered prophylactically or used soon after symptom onset (Casadevall & Pirofski, 2003; Casadevall & Scharff, 1994), providing the only short-term strategy to provide immediate immunity to susceptible individuals, which is paramount in the case of COVID-19.

Despite its long history of use in the treatment of infectious diseases (Luke *et al.,* 2006; Soo *et al.,* 2004; Hung *et al.,* 2011; Zhou *et al.,* 2007; Burnouf & Seghatchian, 2014), results from randomized controlled trials involving convalescent plasma transfusion in COVID-19 are mixed (Focosi *et al.* 2020; Rajendran *et al.* 2020). Many factors may have influenced these results, particularly the size of the enrolled population, the antibody titre of the plasma units, and the stage of disease or days from onset of symptoms at the time of plasma transfusion (The RECOVERY Collaborative Group, 2021; Joyner *et al.,* 2021; Casadevall *et al.,* 2020; Cohn *et al.,* 2021). No clinical trials were conducted soon after SARS-CoV-2 infection or before the onset of significant disease.

Although promising, transfusion of convalescent plasmas has important limitations. First, obtaining plasma depends on voluntary donation from convalescent patients. Furthermore, there is great heterogeneity in the potency of antibodies generated between individuals. In this regard, the US regulatory agency, the FDA, has recommended neutralizing antibody titers in convalescent plasma greater than a> 1:320 for use in COVID-19 (Center for Biological Research and Evaluation, 2020). All these factors together lead to a low yield in obtaining the final product for the treatment of infected people.

These disadvantages can be overcome with the use of heterologous antibodies, obtained from the plasma of horses immunized with SARS-CoV-2. Among other aspects, the use of equine immunoglobulins has the advantages of generating antibodies for long periods, since horses receive periodic booster immunizations, large amounts of plasma and greater control in the final homogeneous preparations. The final product is composed of highly purified antibodies, not crude plasma, providing greater safety and efficacy. Furthermore, it is possible to carry out the enzymatic digestion of the immunoglobulin molecule, removing the Fc fraction, without loss of the neutralizing capacity, which is conferred by the Fab portion of the immunoglobulin.

Worldwide, few clinical trials using purified equine anti-SARS-CoV-2 immunoglobulins for the treatment of patients with COVID-19 are ongoing. A phase 2 clinical trial was initiated in October 2020 in Costa Rica, evaluating two different formulations produced from the immunization of horses with viral proteins (http://www.clinicaltrials.gov. Identifier NCT04610502) (León *et al.,* 2020).

A phase 2/3 clinical trial was carried out in Argentina to evaluate the efficacy and safety of a purified Fab fraction of equine hyperimmune serum generated from antigenic stimulation with the RBD SARS-CoV-2 protein. Preliminary results showed that equine antibody therapy was well tolerated, as adverse reactions were mild and moderate, and hospitalized patients showed clinical improvement of SARS-CoV-2 pneumonia, especially those with severe disease (Lopardo *et al.,* 2021). In parallel, two different IgG formulations were prepared from the plasma of horses immunized with S1 or a mixture of recombinant proteins SARS-CoV-2, and both exhibited high neutralizing capacity *in vitro* (León *et al.,* 2021). A clinical trial is ongoing to compare the safety and efficacy of the equine anti-S1 antibody formulation to treat moderate and severe cases of COVID-19.

All these assays used the spike protein or portions of it as antigens to immunize horses, as this is the protein responsible for binding the virus to the human ACE2 receptor (Du *et al.,* 2009). None of these formulations used all of the virus, which could, in theory, raise additional antibodies that could interrupt other viral processes and help fight the SARS-CoV-2 infection.

SARS-CoV-2 belongs to a large family of viruses called coronaviruses. It is a single positive stranded RNA virus (+ssRNA). Coronaviruses have the ability to cause a variety of illnesses in humans, from simple colds to more serious illnesses such as Middle East Respiratory Syndrome (MERS). SARS-CoV-2 is the seventh coronavirus known to be able to infect humans, the rest being 229E, NL63, OC43, HKU1, MERS-CoV and the original SARS-CoV.

Like the strain that caused the SARS outbreak in 2003, SARS-CoV-2 is a member of the sub-genus Sarbecovirus (line B betacoronavirus). Its RNA sequence is approximately 30,000 nucleobases in length. However, SARS-CoV-2 is the only coronavirus to incorporate a polybasic cleavage site, a feature known to increase the pathogenicity and reproductive rate of other viruses.

From a sufficient number of sequenced genomes, it is possible to reconstruct the phylogenetic tree of the mutations history of a virus family. On January 12, 2020, five SARS-CoV-2 genomes were isolated in Wuhan. As of January 30, 2020, 42 genomes were known. A phylogenetic analysis of these samples disclosed that up to seven mutations were related to a common ancestor, meaning that the first infection in humans occurred in November or December 2019. As of March 13, 2020, 410 SARS-CoV-2 genomes were sampled and publicly available.

On February 11, 2020, the International Committee on Taxonomy of Viruses announced that, in accordance with current rules that determine hierarchical relationships among coronaviruses based on conserved nucleic acid sequences, the differences between SARS-CoV-2 and the SARS-CoV responsible for the SARS outbreak were insufficient to be classified as two different viral species. Thus, SARS-CoV-2 was classified as a strain of coronaviruses associated with acute respiratory syndrome (SARS-CoV).

Each SARS-CoV-2 virion measures approximately 50-200 nanometers in diameter. Like other coronaviruses, SARS-CoV-2 has four structural proteins, known as S (spike), E (envelope), M (membrane) and N (nucleocapsid) proteins. Protein N contains the RNA genome and together the proteins S, E and M create the viral envelope. Protein S is the protein that allows the virus to attach to the cell membrane of a host cell.

### Gamma radiation

Radiant energy for sterilizations has been successfully applied in recent years. Food products, cosmetics, disposable surgical materials, vaccines, serums and blood products and others are decontaminated by radiation.

Electromagnetic energy derived from cobalt is the one that is most frequently used in the sterilization of the materials mentioned. This type of energy, such as gamma radiation, does not heat the product and can therefore also be applied to biological products which, in most cases, are susceptible to relatively high temperatures.

The sterilization of therapeutic serums and some vaccines without the addition of particulate adjuvants is usually done by filtration. Depending on the type of product and the sterilizing filters used, the process is relatively time-consuming and, invariably, volume losses occur, which are sometimes considerable. Due to human failures or other causes, after filtration relatively frequent contaminations are observed that either condemn the product definitively or, at least, force a new filtration and, consequently, increase the loss in volume. In the case of vaccines with added particulate adjuvants, all work is carried out under sterile conditions. In this case, when contamination occurs, the product cannot be recovered.

Gamma radiation, due to its high penetration power, sterilizes products after bottling and even after being packed in usual packaging.

The state of the art does not provide sufficient information on the use of this type of energy in the sterilization of a biological product, such as hyperimmune serums or vaccines. There are practically no doubts about the action of sterilizing gamma rays, but rather about the reduction of biological activity, by destroying the active principles of these products.

Patent application BR 11 2013 001946 8, filed on August 4, 2011, published on May 24, 2016; holder: MERCK SHARP & DOHME CORP. (US), entitled: "INACTIVATED VARICELLA ZOSTER VIRUS, PHARMACEUTICAL COMPOSITION, METHOD FOR PREPARING AN INACTIVATED VARICELLA ZOSTER VIRUS, VACCINE, AND, METHOD FOR THE TREATMENT OF HERPES ZOSTER IN AN INDIVIDUAL" provides an inactivated varicella zoster virus (VZV) and compositions and vaccines comprising said inactivated VZV, and, that VZV infectivity is not detectable, and, wherein the inactivated VZV induces an immune response against VZV when administered to a patient. In embodiments of the compositions described herein, the VZV is inactivated with gamma radiation. Patent application BR 11 2013 001946 8 also provides a method for preparing an inactivated VZV vaccine, the method comprising performing gamma radiation on a sample comprising a VZV by using about 5 kGy to about 50 kGy of gamma radiation. A method for the treatment or immunization against Hz or other disease associated with VZV reactivation is also provided by patent application BR 11 2013 001946 8, the method comprising administering to a subject a vaccine or pharmaceutical composition comprising a therapeutically effective amount of an inactivated VZV and a pharmaceutically acceptable carrier, wherein the VZV is inactivated by gamma irradiation.

Patent application BR 11 2013 001946 8 discloses that the VZV (Varicella Zoster Virus) is inactivated with gamma radiation. The method comprising performing gamma radiation on a sample comprising a VZV by using about 5 kGy to about 50 kGy of gamma radiation.

It is important to note that in the case of viruses, they are generally very resistant to ionizing radiation and each virus has to be studied independently (including each viral strain within the same type of virus), first to determine its radiosensitivity, known as D10 (sufficient dose to reduce the microbiological load by 1 log), and from this determination, the total viral load and taking into account the desired safety level (SAL) it is possible to determine the desired absorbed dose to safely inactivate all material.

In the case of most viruses, this final dose ends up being very high and, therefore, making its inactivation unfeasible, without changing the characteristics of the material to be sterilized or in the case of focusing on the virus itself, for use in vaccines or serum therapy, destroying its tertiary structure, losing its immunogenic characteristics.

US Patent Application US 2006121580, filed on January 20, 2006, published on June 8, 2006, in the name of CRUCELL, entitled "BINDING MOLECULES AGAINST SARS-CORONAVIRUS AND USES THEREOF" refers to binding molecules that specifically bind SARS-CoV, nucleic acid molecules encoding the binding molecules, compositions comprising the binding molecules, and methods for identifying or producing the binding molecules. The binding molecules are capable of specifically binding to SARS-CoV and can be used in the diagnosis, prophylaxis and/or treatment of a condition resulting from SAR.

US Patent Application US 2006121580 discloses the use of about 45 kGy of gamma radiation to inactivate SARS-COV. The present application discloses a process for producing an antigen corresponding to SARS-CoV-2 viruses inactivated by gamma irradiation, preferably using about 15 kGy of gamma radiation.

The inventors of this application emphasize that, in the case of most viruses, this final dose (45 kGy) ends up being very high and, therefore, making its inactivation unfeasible, without changing the characteristics of the material to be sterilized or in the case of focus on the virus itself, to be used in vaccines or serum therapy, destroying its tertiary structure, losing its immunogenic characteristics.

International Patent Application WO2009106629, filed on February 27, 2009, published on September 3, 2009, in the name of UNIVERSITEIT GENT, entitled "VIRAL INACTIVATION PROCESS" refers to methods for determining the effect of a procedure of viral inactivation on the antigenicity of the inactivated virus. In particular, for a virus that is a member of the *Arteriviridae* or *Coronaviridae* or *Asfarviridae* family, in particular, for the porcine reproductive and respiratory syndrome virus (PRRSV). International Patent Application WO2009106629 further provides methods for determining the antigenicity of an inactivated virus, as well as methods for screening antiviral compounds using any of the methods mentioned above. Methods for using the inactivated and immunogenic virus thus obtained, in particular in the manufacture of a vaccine, are also provided by International Patent Application WO2009106629.

Said International Patent Application WO2009106629 discloses that viral inactivation can be done by temperature, gamma irradiation, UV light and BEI for members of the *Arteriviridae* or *Coronaviridae* or *Asfarviridae* family. Said document is completely silent as to the amount of gamma rays used and neither specific SARS-CoV-2.

US Patent 8,106,170, filed on November 10, 2005, published on May 18, 2006, and granted on June 31, 2012 in the name of CRUCELL HOLLAND BV, entitled "COMPOSITIONS AGAINST SARS-CORONAVIRUS AND USES THEREOF" refers to compositions of binding molecules that specifically bind to a coronavirus, such as SARS-CoV, and are capable of neutralizing an infection caused by the virus. The compositions are suitable for diagnosing, preventing and/or treating a condition resulting from a coronavirus such as SARS-CoV.

U.S. Patent US 8,106,170 discloses compositions of binding molecules that specifically bind to a coronavirus, such as SARS-CoV, and are capable of neutralizing an infection caused by the virus. Inactivation can be done by gamma irradiation at 45 kGy. Once again it is highlighted that, in the case of most viruses, this final dose (45 kGy) ends up being very high and, therefore, making its inactivation unfeasible, without changing the characteristics of the material to be sterilized or in case of focusing on the same virus, to be used in vaccines or serum therapy, destroying its tertiary structure, losing its immunogenic characteristics.

US Patent 8,506,968, filed on December 28, 2009, published on June 23, 2011, and granted on August 13, 2013 in the name of LILLY CO ELI, entitled "SARS VACCINE COMPOSITIONS AND METHODS OF MAKING AND USING THEM" describes a composition and method for reducing the occurrence and severity of infectious diseases, especially infectious diseases such as SARS, in which infectious viral organisms containing lipids are found in biological fluids such as blood. U.S. Patent 8,506,968 employs solvents useful to extract lipids from the lipid-containing infectious viral organism, thereby creating partially delipidated immunogenic modified viral particles with reduced infectivity. U.S. Patent US 8,506,968 provides delipidated viral vaccine compositions, such as therapeutic vaccine compositions, comprising such modified partially delipidated viral particles with reduced infectivity, optionally combined with a pharmaceutically acceptable carrier or an immunostimulant.

U.S. Patent US 8,506,968 discloses a composition and method for reducing the occurrence and severity of infectious diseases, especially infectious diseases such as SARS. Inactivation can be done by gamma irradiation at 50 kGy. It is known that, in the case of most viruses, this final dose (50 kGy) ends up being very high and, therefore, making its inactivation unfeasible, without changing the characteristics of the material to be sterilized or in the case of focusing on the virus itself, to use in vaccines or serum therapy, destroying its tertiary structure, losing its immunogenic characteristics.

The article by Christin Scheller et al., entitled: "PHYSICOCHEMICAL PROPERTIES OF SARS-COV-2 FOR DRUG TARGETING, VIRUS INACTIVATION AND ATTENUATION, VACCINE FORMULATION AND QUALITY CONTROL". Electrophoresis; 41(13-14):1137-1151, published on June 8, 2020 discusses the material properties of the severe acute respiratory syndrome coronavirus 2 (SARS-CoV-2) and its proteins. In said article it is discussed that gamma radiation can inactivate biological material by the displacement of electrons, the breaking of covalent bonds or indirect damage through free radicals formed after the breaking of covalent bonds. In this study, a SARS-CoV-1 solution (400 µL of 106.33 TCID50 per mL) was subjected to gamma radiation (30, 50, 100 and 150 Gy) from a 60Co source. However, no significant effect on virus infectivity was observed after 15 minutes of exposure.

Basically, Christin Scheller's article discloses inactivation of SARS-COV-1 by the use of 30, 50, 100 and 150 KGy. However, the article itself also discloses that: *"However, since seemingly small differences can have strong effects, for example, in immunologically relevant epitope configurations, unassessed knowledge transfer from SARS-CoV-1 to SARS- CoV-2 cannot be advised".*

The article by Feldmann F. et al., entitled: "GAMMA IRRADIATION AS AN EFFECTIVE METHOD FOR INACTIVATION OF EMERGING VIRAL PATHOGENS", Am J Trop Med Hyg, published in May 2019; 100 (5) :1275-12770 comments that gamma irradiation using a cobalt-60 source is a commonly used method for inactivating infectious specimens to be safely handled in subsequent laboratory procedures. In said article, irradiation doses to safely inactivate liquid protein specimens containing different emerging/re-emerging viral pathogens known to cause neglected tropical diseases and other diseases of regional or global importance to public health were determined. Using a representative *arenavirus, bunyavirus, coronavirus, filovirus, flavivirus, orthomyxovirus, and paramyxovirus,* these enveloped viruses were found to differ in their susceptibility to irradiation treatment with adsorbed doses for inactivation of a target dose of 1 × 10⁶ 50% of culture of infectious tissues dose (TCID50)/mL ranging from 1 to 5 MRads.

The article by Feldmann F. discloses *inactivation by gamma irradiation of several virus species, including SARS-CoV (Tor 2 strain). Uses between 10 to 50 kGy for inactivation by gamma irradiation. As mentioned above, in the case of viruses, they are generally very resistant to ionizing radiation and each virus has to be studied independently (including each viral strain within the same type of virus)* . *With SARS-CoV-2, the inventors of the present application were able to determine a high radiosensitivity, enabling its inactivation, even at high concentrations without changing its tertiary structure, maintaining its immunogenicity, as well as maintaining the biological activity of infected plasmas and cell supernatants.*

The article by Leung Anders et al., entitled: "IN VITRO INACTIVATION OF SARS-COV-2 USING GAMMA RADIATION" - Appl. Biosafety; published on July 30, 2020, discloses that coronavirus 2 (SARS-CoV-2) is classified as a Risk Group 3 pathogen; propagation work with this live virus should be conducted in biosafety level 3 (BSL-3) laboratories. However, the inactivated virus can be safely handled in BSL-2 labs. Gamma irradiation is one of the methods used to inactivate a variety of pathogens, including viruses. The article discloses inactivation of SARS-COV-2 by gamma radiation using 10 kGy of gamma radiation.

In the article by Leung Anders, a gamma irradiation process for the inactivation of SARS-CoV-2 virus is described in order to find the dose necessary for the sterilization of the virus, without compromising its detection by polymerase chain reaction (PCR). The article uses the same technique used for the inactivation of SARS-CoV-2 for the purpose of producing the antigen used in the immunization of horses, to obtain the serum. However, the analysis carried out to verify the material inactivation procedure aimed at two different objectives, used different methods and led to different conclusions. The conclusion of the article on the integrity of the SARS-CoV-2 genetic material does not enable the inactivated virus to be used as an antigen, since there is no biochemical analysis of the integrity or immunogenicity and antigenicity of viral proteins, which is essential for an antigen to be used for the production of hyperimmune serum.

In the present patent application, data from analyzes carried out to verify the quality of viral proteins after inactivation are presented, including proof that the material, later used for the immunization of animals, was able to generate neutralizing antibodies. Viral inactivation by irradiation alone does not guarantee *a priori* that the material is sufficiently immunogenic, which is easily verified by exposing the same sample to different amounts of radiation, resulting in different levels of antigen recognition, as a result of partial destruction and dose-dependent on viral proteins by exposure to radiation. Finally, the material used in the irradiation process described in the article was not purified or formulated, being just a collection of infected cell culture supernatant. In the case described in the present patent application, the material was irradiated purified and formulated in a preservation buffer. As in any other inactivation method, the concentrations of the active microorganism influence the irradiation performance, as well as the concentrations of other by-products of material production in cells (cell lysates composed of proteins and cellular DNA/RNA). Therefore, it cannot be assumed that the irradiated material without the preservation buffer or in its unpurified form will be able to maintain the same protein characteristics of the irradiated material in the pure form and in the preservation buffer.

As can be seen, no prior art document describes or suggests a process for the production of an antigen, corresponding to the SARS-CoV-2 viruses, inactivated by gamma ray inactivation as disclosed by the present application.

### SUMMARY OF THE INVENTION

To solve the aforementioned problems, the present invention will provide significant advantages in relation to the production processes of an antigen, corresponding to the inactivated SARS-CoV-2 viruses, using gamma ray inactivation, enabling an increase in its performance and presenting a more favorable cost/benefit ratio.

Based on all the information mentioned above and considering the urgency of a specific treatment for COVID-19, the aim of the present application was to develop an anti-SARS-CoV-2 equine immunoglobulin F(ab')2 for the treatment de COVID-19, emphasizing that it is a known, tested and approved final product, with wide application in the treatment of other pathologies. Thus, in the present application, the inventors describe the development of anti-SARS-CoV-2 F(ab')2 equine immunoglobulins using a newly developed SARS-CoV-2 viral antigen that has been purified and inactivated by radiation. Cellular and preclinical assays have shown that the serum can successfully neutralize the virus, is safe in animal models, and is able to reduce disease severity in a hamster model with SARS-CoV-2 infection and disease.

The present invention relates to a process for producing an antigen, corresponding to the inactivated SARS-CoV-2 viruses comprising the steps of cultivation and multiplication of the SARS-CoV-2 virus in mammalian cells; collection; virus purification; viral stabilization, and viral inactivation, wherein viral inactivation is by gamma irradiation.

In a second embodiment, the present invention relates to tests for detection of the active virus and verification of inactivation in vaccine samples, kits, culture supernatants, cell lysates or whole cells.

In a third embodiment, the present invention refers to the use of the antigen, corresponding to the inactivated SARS-CoV-2 viruses obtained by the process of the present invention for the preparation of a new vaccine.

In a fourth embodiment, the present invention refers to the use of the antigen, corresponding to the inactivated SARS-CoV-2 viruses obtained by the process of the present invention for the production of hyperimmune plasma in horses for serum therapy.

In a fifth embodiment, the present invention refers to the use of the antigen, corresponding to the inactivated SARS-CoV-2 viruses obtained by the process of the present invention in different animal species for the production of antibodies/inputs to research and serodiagnostic kits.

In a sixth embodiment, the present invention refers to the use of the antigen, corresponding to the inactivated SARS-CoV-2 viruses obtained by the process of the present invention for the preparation of a composition intended for immunization for the protection of the immunized individual or animal.

In a seventh embodiment, the present invention relates to an antigenic composition comprising the inactivated SARS-COV-2 virus obtained by the process of the present invention.

In an eighth embodiment, the present invention relates to a vaccine composition comprising hypermune serum for serum therapy.

In a ninth embodiment, the present invention relates to a diagnostic kit with the antigenic composition comprising the inactivated SARS-CoV-2 virus obtained by the process of the present invention and a diluent.

### BRIEF DESCRIPTION OF THE FIGURES

The structure and operation of the present invention, together with additional advantages thereof, can be better understood by reference to the attached drawings and the following description:

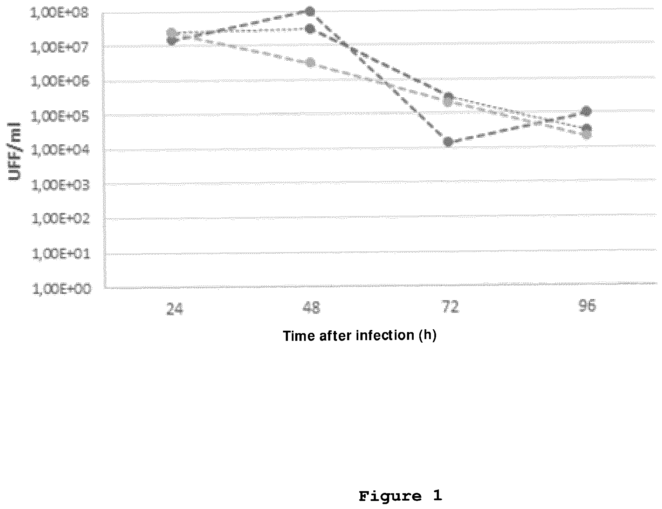

Figure 1 shows the Production of viruses with different MOI (0.1, 0.05 and 0.01) and different times after infection;

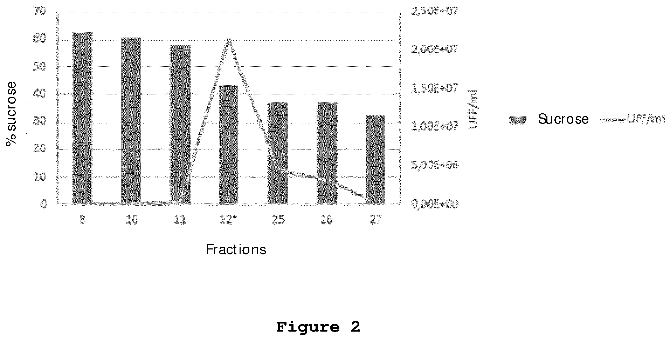

Figure 2 shows the sucrose gradient ultracentrifugation carried out in a zonal rotor. Fraction 12* corresponds to fractions 12 to 24 (sucrose 55 to 35%) containing the virus peak.

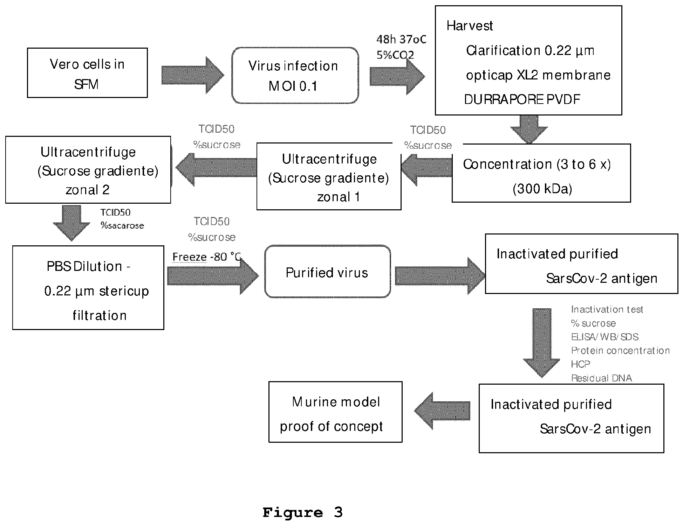

Figure 3 shows a detailed flowchart of the steps in the process of producing an antigen, corresponding to the inactivated SARS-CoV-2 viruses;

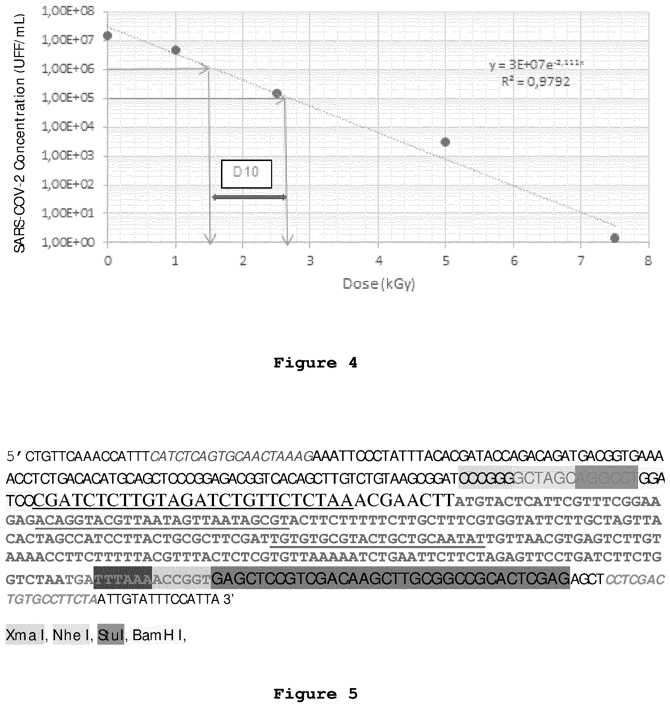

Figure 4 shows the determination of D10 (dose required to inactivate 1 log of the microbiological population), in which the viral concentration: batch 1 fourth pass ~ 107, Irradiation doses (seven samples) predicting a D10 between 2 and 2.5 kGy: non-irradiated; 1 kGy; 2.5 kGy; 5 kGy; 7.5 kGy; 10 kGy and 15 kGy; Irradiation carried out at GammaCell starting on April 30^{th} and ending on May 1^{st}, 2020.
Figure 5 shows a DNA fragment containing the sequence of protein E (target prot Charit6) and region of the fragment corresponding to the subgenomic RNA that was synthesized;

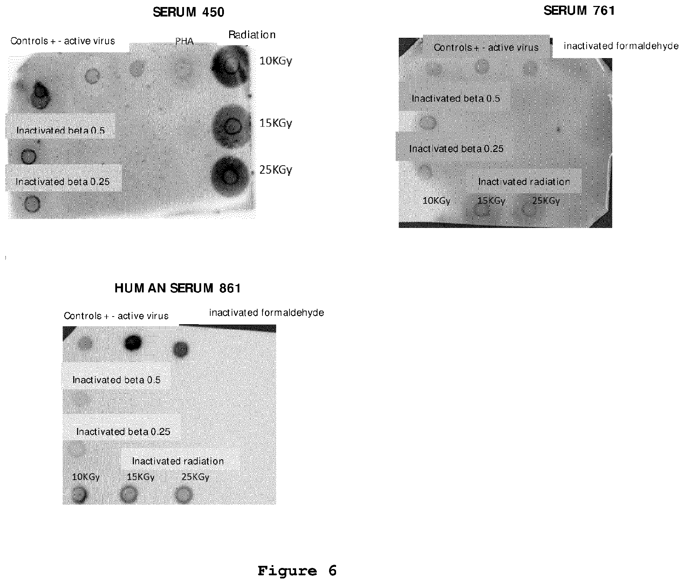

Figure 6 shows the inactivation of serum 450, serum 761 and human serum 861;

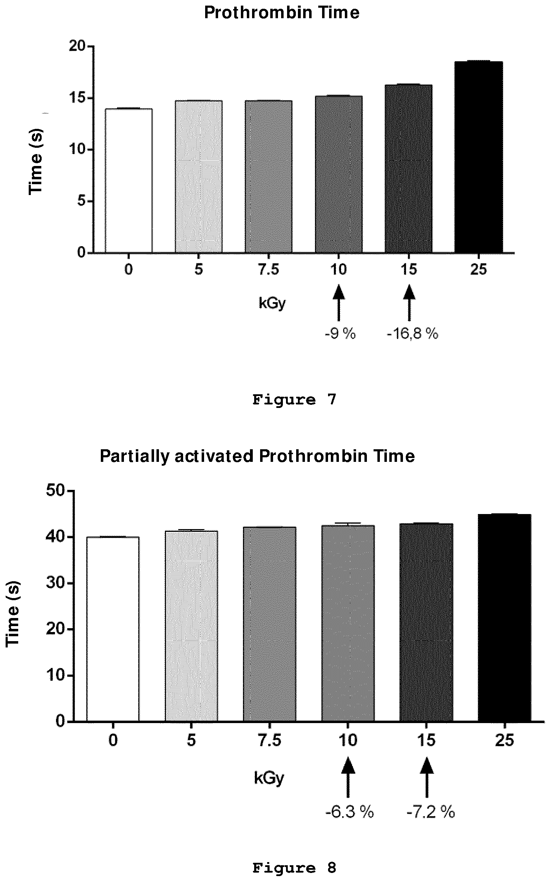

Figure 7 shows the result of the prothrombin time, where the platelet poor plasma (100 µL) was exposed to different doses of ionizing radiation (5 to 25 kGy). After incubation, 100 µl of thromboplastin was added and the clotting time measured. Columns represent the average of triplicates ± DP. (- %) = percentage of coagulation enzyme activity;
Figure 8 shows the partially activated thromboplastin time, where the platelet-poor plasma (100 µL) was exposed to different doses of ionizing radiation (5 to 25 kGy). After incubating the PPP with cephalin and ellagic acid, 100 µL of CaCl₂ were added. Columns represent the average of triplicates ± DP. (- %) = percentage of coagulation enzyme activity;

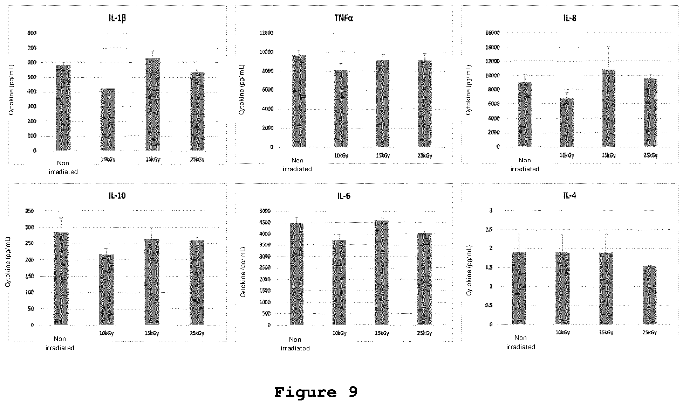

Figure 9 shows the quantification of inflammatory cytokines using Multiplex kits (Merck) in the Luminex equipment in THP-1 cell supernatants after irradiation with doses of 10kGy, 15kGy and 25kGy. The cytokines IL-6, Il-8, IL-1β, IL-4, IL-10 and TNFα were measured.

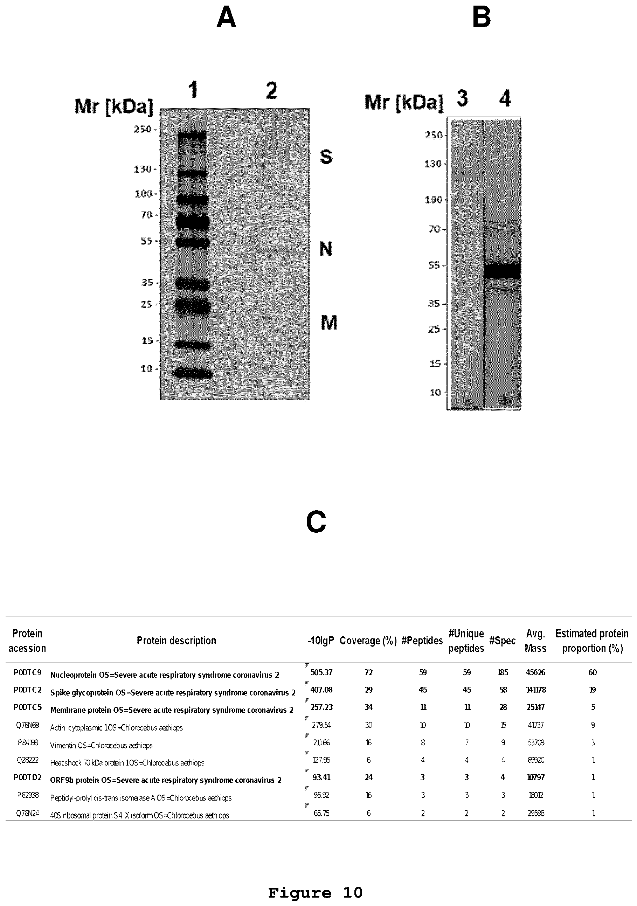

Figure 10 shows the analysis of purified and inactivated SARS-CoV-2 virus. [A] SDS-PAGE: 4-15% gradient SDS-PAGE gel under non-reducing conditions and silver stained. (1) LMW -10 to 250 kDa; (2) SARS-CoV-2 purified and inactivated. [B] Western blot: Virus samples, separated on 4-15% gradient SDS-PAGE, were transferred to nitrocellulose membranes and incubated with anti-SARS-CoV-2 S protein (lane 4) monoclonal antibodies (MoAb) or anti-SARS-CoV-2 N protein (lane 5), LMW -10 to 250 kDa (lanes 3 and 6). Membranes were incubated with specific conjugated anti-rabbit IgGs (1:5000) and reactions were disclosed by the chemiluminescent substrate Super signal West pico. [C] Protein identification by tandem mass spectrometry (LC-MS/MS) analysis of purified inactivated SARS-CoV-2 antigen. Only proteins with at least 1 single peptide, with a -log10P score> 20 and a false discovery rate <1% were accepted for identification and are represented in the table. The proportion of proteins from proteins was estimated based on the mass spectra count of each identified protein.

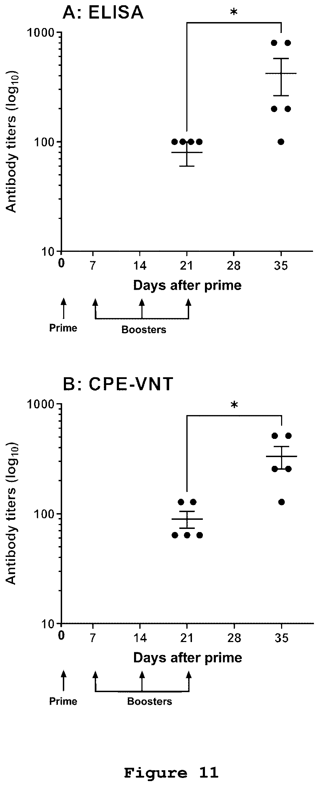

Figure 11 shows the immunogenicity of purified and inactivated SARS-CoV-2 virus in murine model. [A] ELISA plates were coated with 100 µL of purified and inactivated SARS-CoV-2 (4.2 × 104 virus / mL) and incubated with increasing dilutions of non-immune or experimental sera obtained from BALB / c mice and incubated with anti - mouse IgG conjugated to HRPO. The reaction was carried out with the addition of TMB substrate, and spectrophotometric readings were taken at λ 450 nm. The titer was established as the highest serum dilution at which the measured absorbance was twice as high as that determined for normal mouse serum (control group). [B] Sera from mice immunized with purified and inactivated SARS CoV-2 antigen were also tested in CPE-VNT. The neutralizing titer was defined by the inverse of the highest serum dilution that blocks viral replication. Statistical analyzes were carried out by using t-student followed by the Mann Whitney test (*p≤ 0.05).

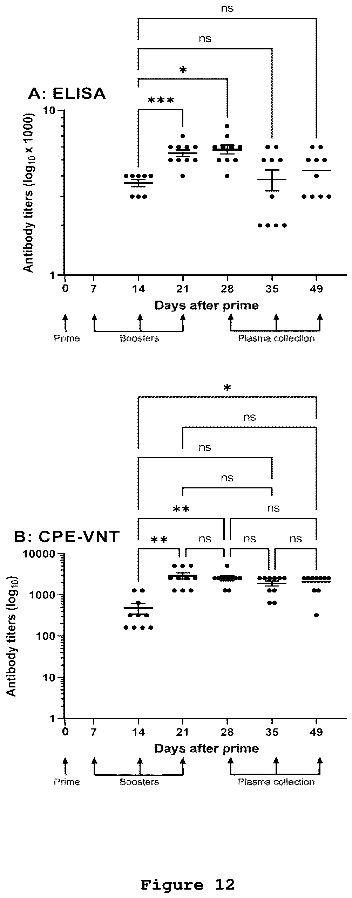

Figure 12 shows anti-SARS-CoV-2 horse hyperimmune plasma titers. [A] ELISA plates were coated with 100 µL of purified and inactivated SARS-CoV-2 (4.2 × 104 virus / mL) and incubated with increasing dilutions of pre-immune or experimental sera obtained from horses hyperimmunized with the inactivated SARS-CoV-2. Then, plates were incubated with anti-horse HRPO-conjugated IgG. The reaction was carried out with the addition of TMB substrate, and spectrophotometric readings were taken at λ 450 nm. The titer was established as the highest serum dilution at which the measured absorbance was twice as high as that determined for the pre-immune serum (control group). [B] Sera from horses immunized with purified and inactivated SARS CoV-2 antigen were also tested in CPE-VNT. The neutralizing titer was defined as the inverse of the highest serum dilution that blocks viral replication. Statistical analyzes were carried out using t-student followed by the Mann Whitney test (*p≤ 0.05).

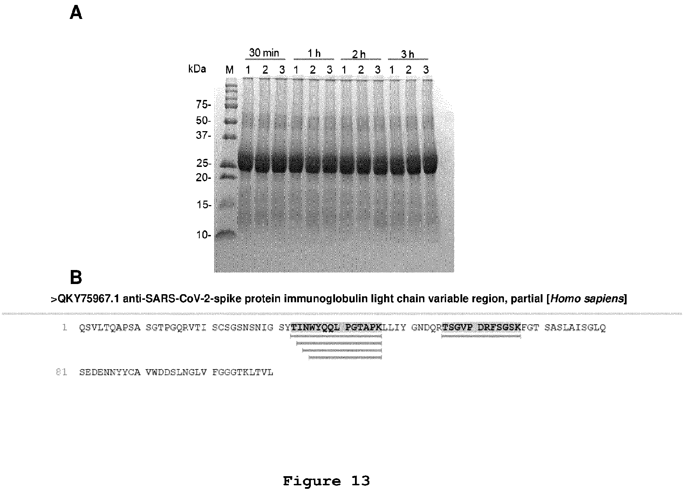

Figure 13 shows the hyperimmune serum protein profile of horse anti-SARS-CoV-2 and identification of specific anti-peak immunoglobulin by LC-MS/MS analysis. [A] SDS-PAGE protein profile: 1, 2 and 3 correspond to samples from lots 0001, 0002 and 0003, respectively, the incubation times at 37 °C are shown above the gel and the molecular mass markers are indicated on the left side. [B] Example of protein identification from specific anti-Spike immunoglobulin mass spectrometry. The same sequence was identified in all analyzed batches;

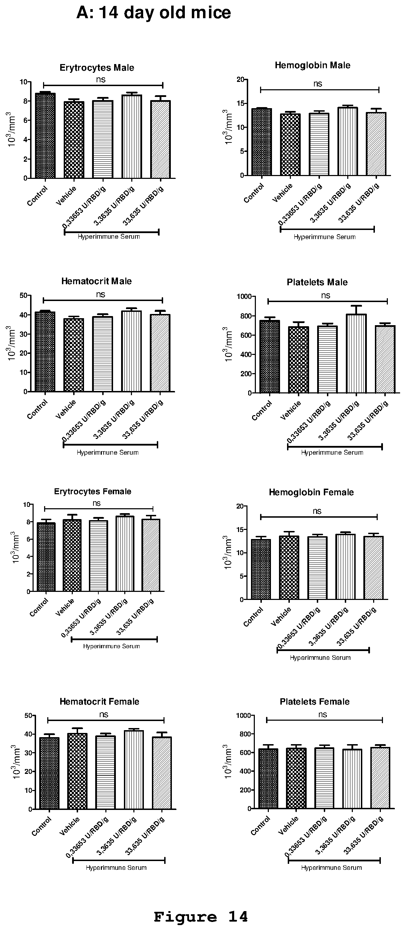

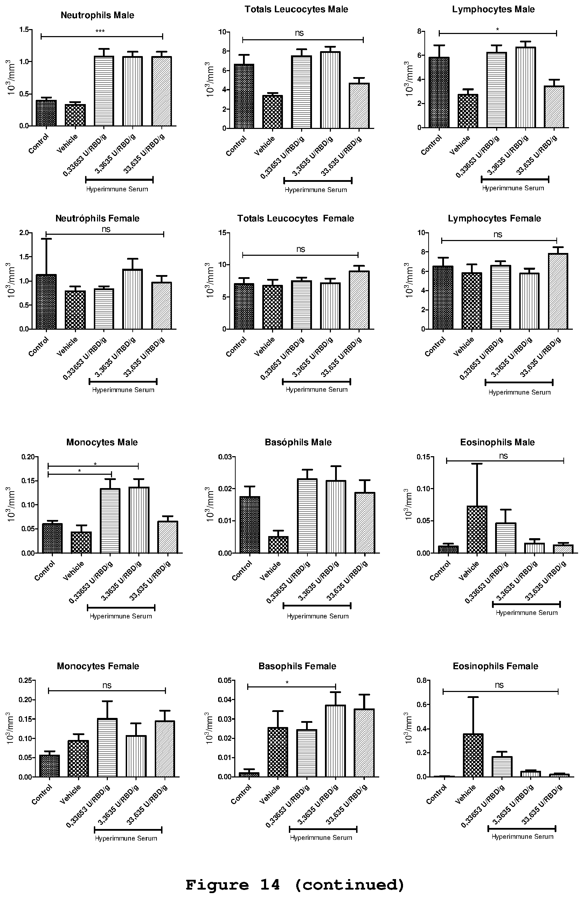

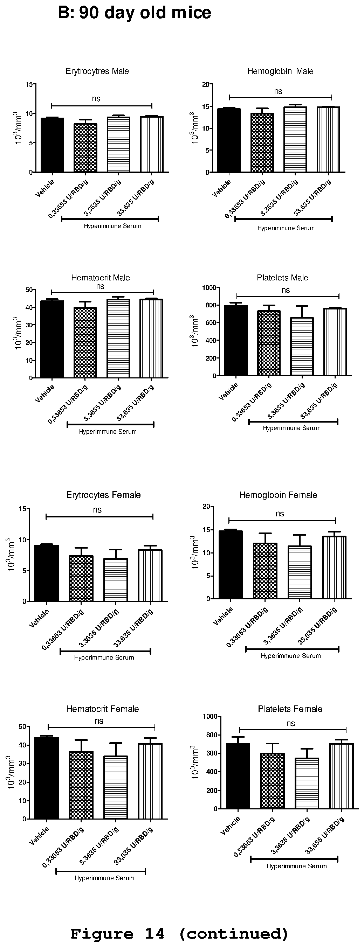

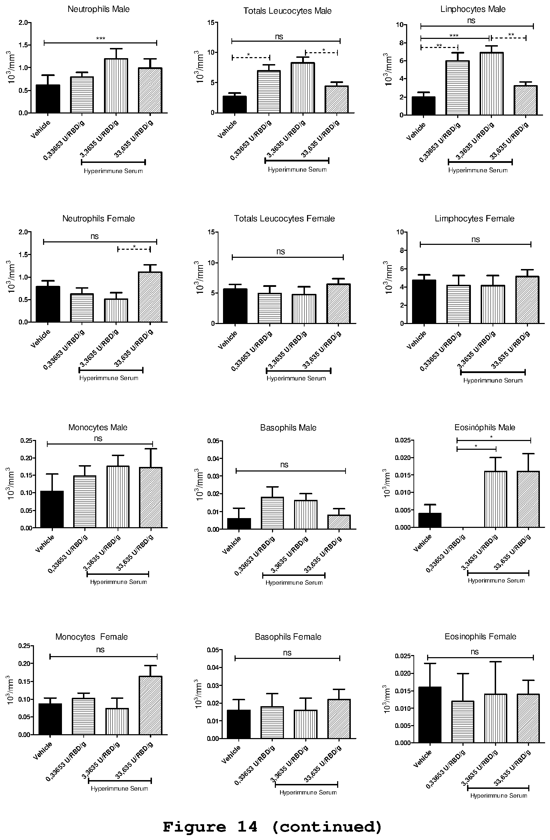

Figure 14 shows the toxicity and safety: hematological analysis. Figure 14A shows in 14-day-old mice and Figure 14B shows in 90-day-old mice;

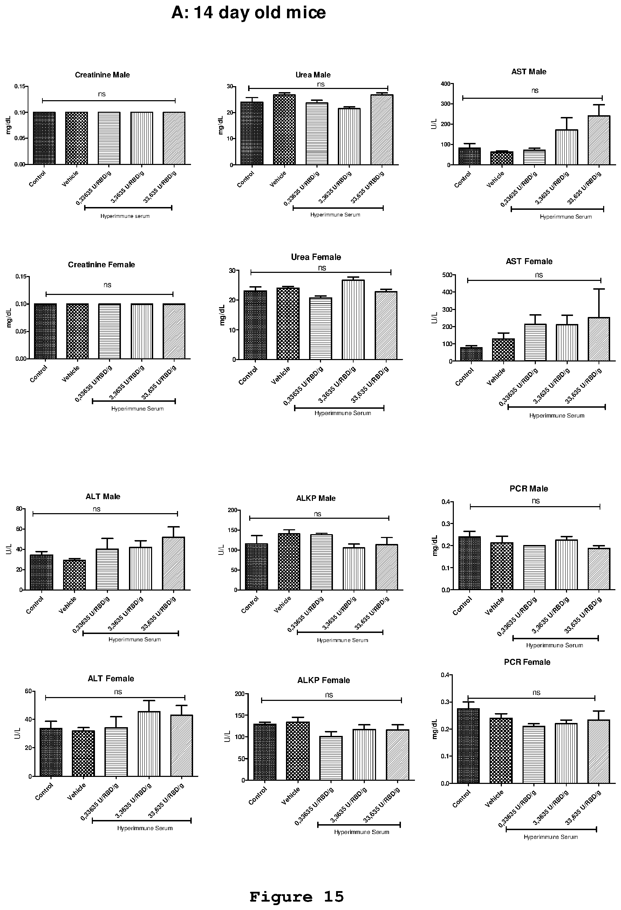

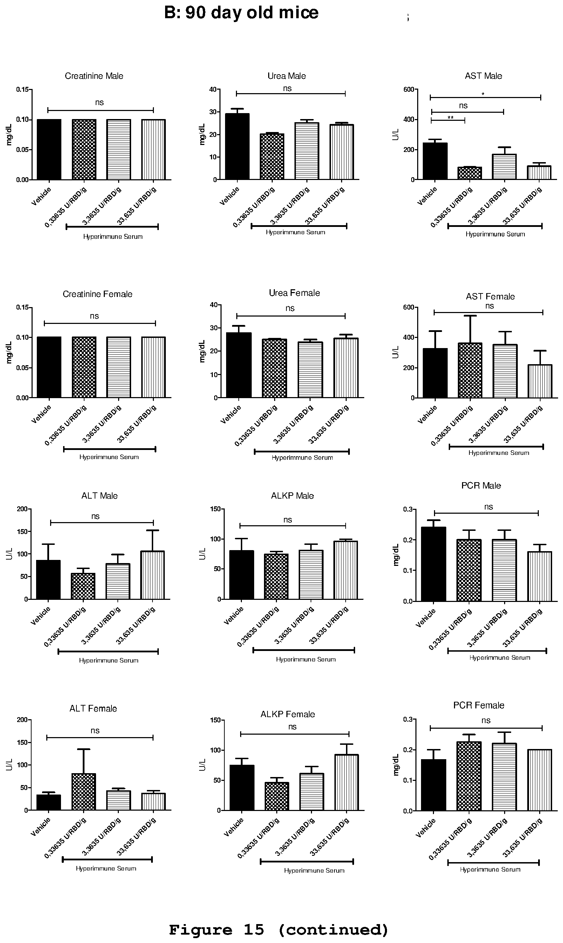

Figure 15 shows the toxicity and safety: biochemical analysis. Figure 15A shows in 14-day-old mice and Figure 14B shows in 90-day-old mice;

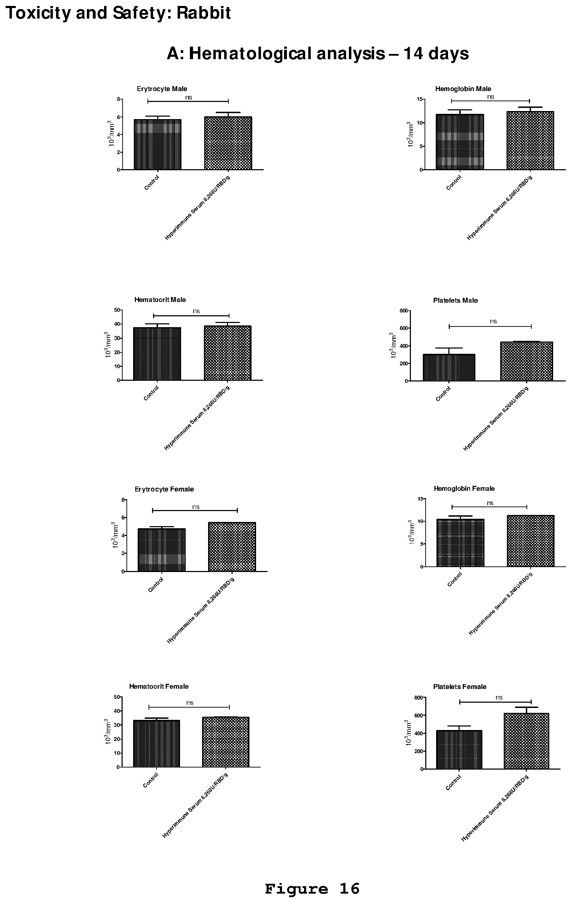

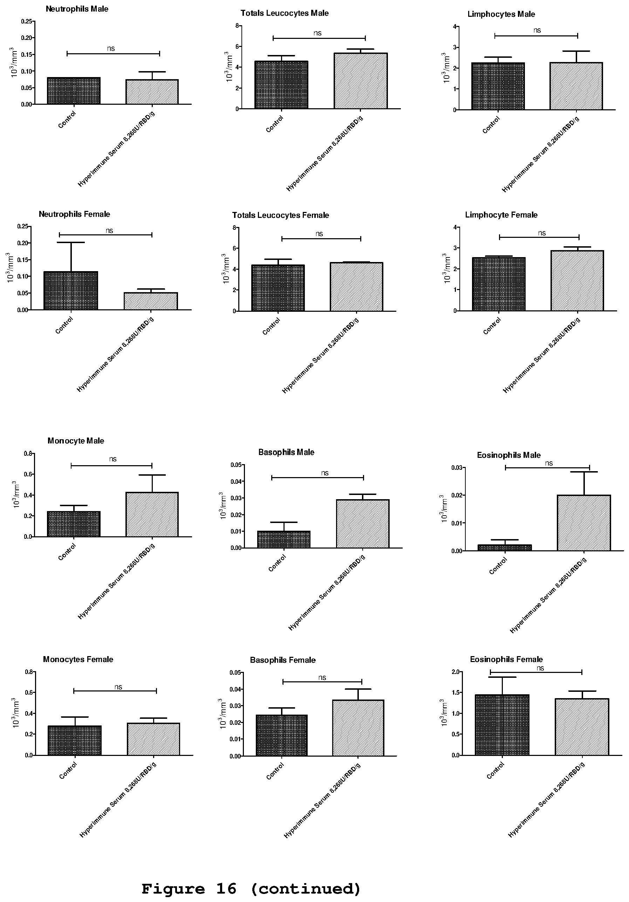

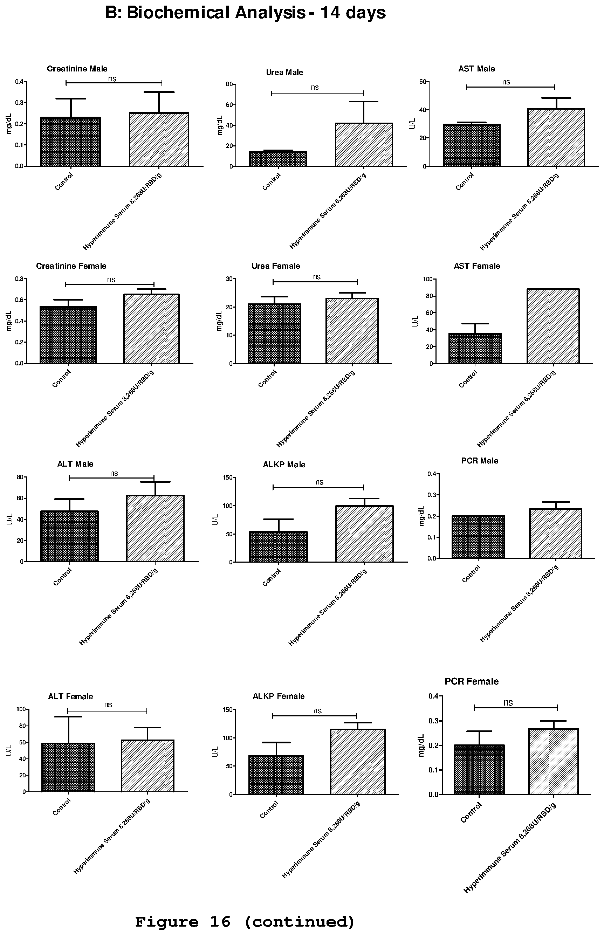

Figure 16 shows the toxicity and safety profile. A - Hematological analysis in 14-day-old rabbits and B - Biochemical analysis in 14-day-old rabbits;

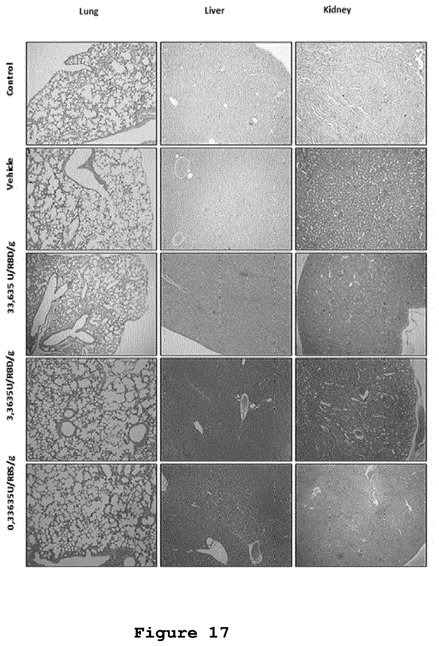

Figure 17 shows the histopathological analysis carried out 14 days after the administration of anti-SARS-CoV-2 serum in toxicity and safety test in mice. Anti-SARS-CoV-2 serum concentrations of 33.635 U / RBD / g, 3.365 U / RBR / g and 0.3365 U / RBD / g were administered intravenously. No alterations were observed in the structures of the lung, liver and renal parenchyma. No inflammatory, degenerative or fibrotic processes was found. 40X magnification;

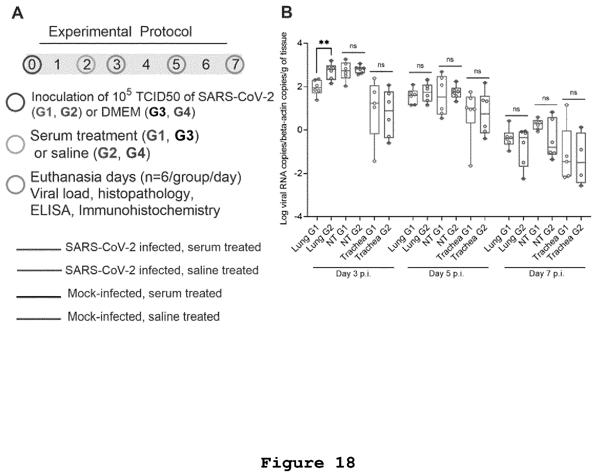

Figure 18 shows the Challenge Test in golden Syrian hamsters. [A] Study drawing - schedule of infection of animals with SARS-CoV-2 followed by treatment with anti-SARS-CoV-2 equine serum. [B] SARS-CoV-2 RNA copies as measured by RT-qPCR per β-actin RNA copy number per gram of tissue. Green: G1 animals (n = 6 per group), i.e., serum treated SARS-CoV-2 infected animals; Red: G2 animals (n = 6 per group), ie untreated SARS-CoV-2 infected animals. p.i. = post-infection; n.s.: not significant (p value > 0.05); ** p value = 0.0043 (day 3 p.i.)

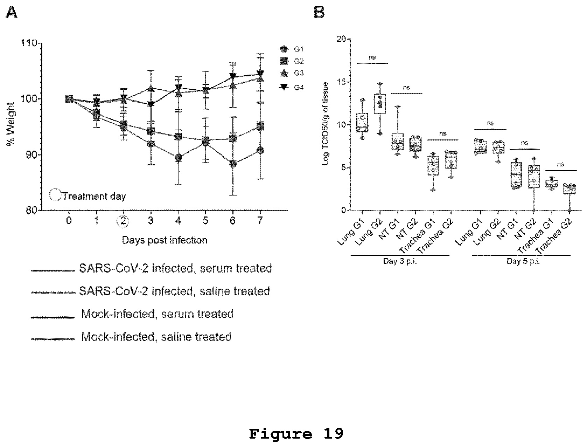

Figure 19 shows the Challenge Test in golden Syrian hamsters. [A] Weight changes observed in animals infected or not with SARS-CoV-2, submitted or not to serological treatment. [B] Viral load expressed as 50% tissue culture infectious dose / gram tissue. Green: G1 animals (n = 6 per group), i.e., serum treated SARS-CoV-2 infected animals; Red: G2 animals (n = 6 per group), ie untreated SARS-CoV-2 infected animals. p.i. = post-infection; n.s.: not significant (p value > 0.05);

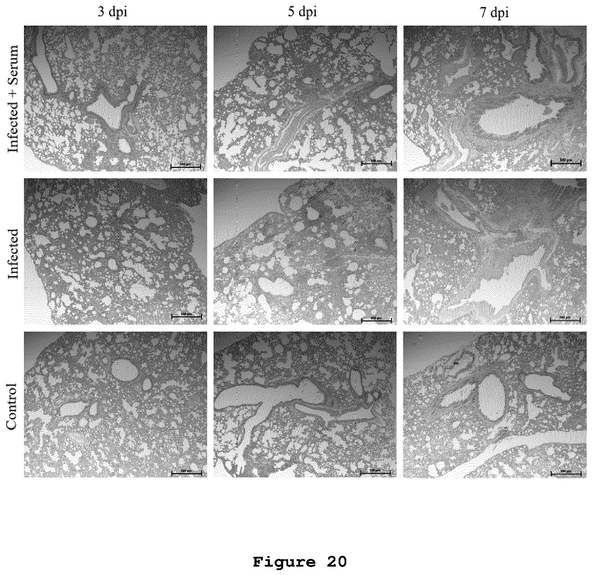

Figure 20 shows the pneumonic pattern of animals infected and treated with anti-SARS-CoV-2 equine serum. Panels show the evolution of the infectious process in the course of experimental infection of the SARS-CoV-2 hamster and treatment with equine serum. Infected and untreated animals show a more accentuated evolution of the pattern of diffuse interstitial pneumonia, especially on the 5^{th} day after infection, compared to animals infected and treated with SARS-CoV-2. Control samples show the normal histological pattern of the tissue. Bar: 500 µm; Hematoxylin and eosin (H&E) staining.

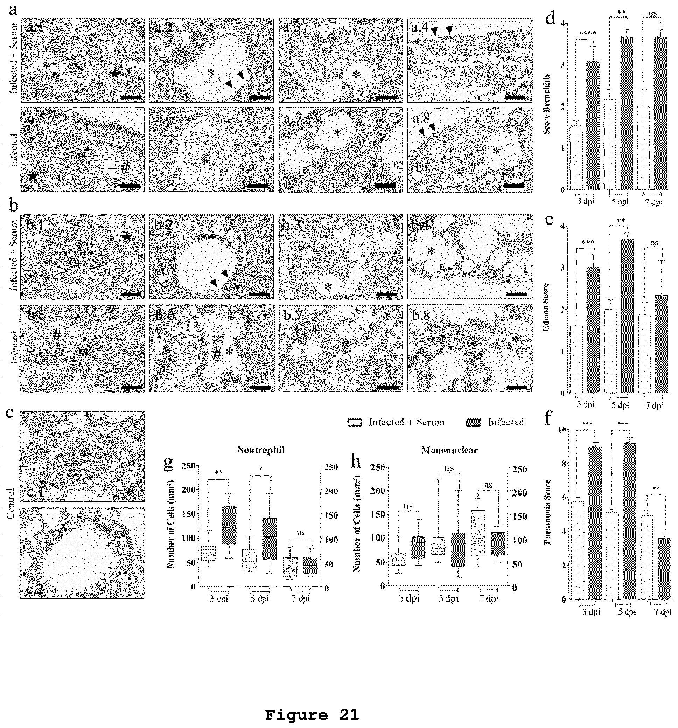

Figure 21 shows the histopathological changes in hamsters infected and treated with anti-SARS-CoV-2 equine serum. Histopathological analysis of vascular (a.1-5: b.1-5: c.1-5), bronchial (a.2-6: b.2-6: c.2-6), alveolar / interstitial (a.3-7: b.3-7: c.3-7) and pleural epithelium (a.4-8: b.4-8: c.4-8) in 3, 5 and 7 days [D1] after the experimental infection, showing bronchopneumonia, with differences in the integrity of the bronchial epithelium (arrows) between treatments. (*) indicates the light of the structures; () the degeneration or not of the epithelium with desquamated cells; () shows swollen perivascular region / leukocyte infiltrates, (He) shows red blood cells in the lumen / interstitium; (Ed) edema formation / hyaline substance and (#) deposit of amorphous substances (c). Blood vessels (b.1) and bronchioles (b.2), normal representative of the uninfected control group. Bronchitis (d), edema (e) and pneumonia score. Leukocytes infiltrate (g) neutrophils and (h) mononuclear kinetics. Data expressed on average + standard deviation, from samples of 6 (six) animals per group, for the scores and 5 (five) fields/images per slide. Data were statistically analyzed using One-Way ANOVA, followed by Tukey's post-test. **** p <0.0001. Bar: 50 pm; Hematoxylin and eosin (H&E) staining.

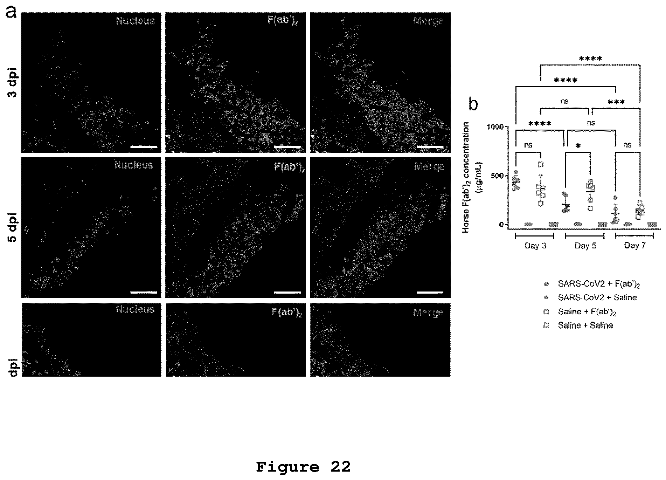

Figure 22 shows antibody detection in the golden Syrian hamster model COVID. (a) F(ab') 2 positive immunofluorescence in the lung in the uninfected group. (b) ELISA plates were coated with 100 µl of rabbit anti-horse IgG and then incubated with a previously prepared standard curve consisting of known concentrations of anti-SARS-CoV-2 horse F (ab') 2, parallel to hamster serum Horse F(ab') 2 in the standard curve or in the samples was then detected by the addition of peroxidase-conjugated anti-horse IgG followed by TMB substrate. Absorbances were taken at 450 nm. Standard curve was linearized by log-log transformation and then submitted to linear regression. The concentration of horse F(ab')2 in hamster serum samples was estimated by interpolating the samples to the standard curve;

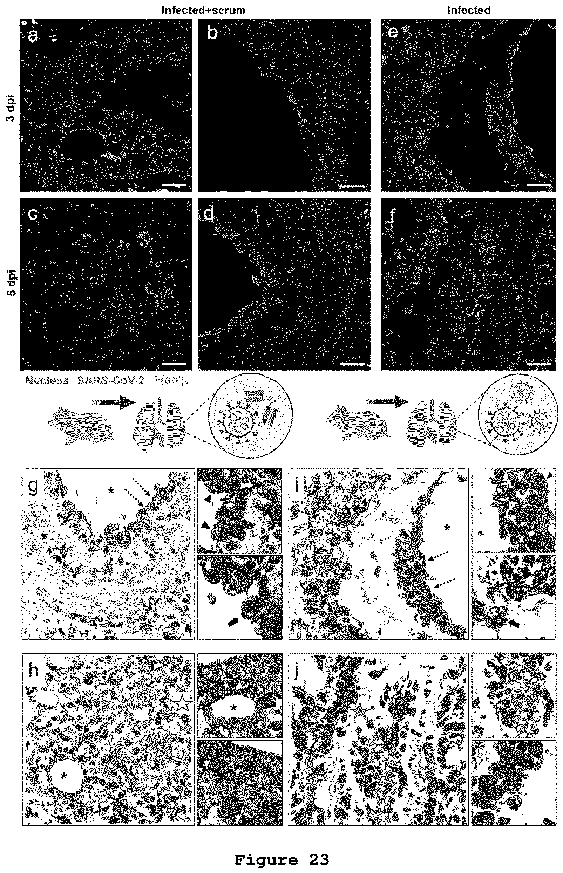

Figure 23 shows the protective effect of anti-SARS-CoV-2 in the lung of the golden Syrian hamster model COVID. (a-b-c-d) Positive immunostaining for Spike protein and F(ab')2 in lung tissue from infected and serum-treated animals. (g-h) 3D analysis showing in details the kinetics and interaction of SARS-CoV-2 with F(ab')2. (e-f) Distribution of SARS-CoV-2 in experimentally infected lung tissue and (i-j) 3D infection kinetics. (*) Light from the bronchioles or alveoli; (yellow star) interstice; (blue star) vascular endothelium; (dotted arrows) indicate the area in greatest magnification to the right; (arrowhead) regions of viral adhesion and antibodies; (arrow): protective effect of F(ab') 2 on an infected or bronchial epithelial cell. Bar: 20 µm.

### DETAILED DESCRIPTION OF THE INVENTION:

The production of SARS-CoV-2 virus can be carried out in mammalian cells. In one context, the cells preferably used are VERO CCL-86 or VERO E6 (*Cercopithecus aethiops*) qualified for the production of immunobiologicals.

Mammalian cells are cultivated in specific culture media, preferably, adapted to growth in fetal bovine serum free medium. In a preferred context, these culture media are VP SFM (Thermo Scientific) and OptiPro (Thermo Scientific) media.

Cells are cultured in adherence to surfaces or cultured in suspension.

The surfaces used can be two-dimensional or three-dimensional, composed of plastic or resins, being flasks, porous or non-porous microcarriers, composed of synthetic or natural fibers, or derivatives of the petrochemical industry. The culture flasks can be type T, "roller" type bottles, fixed or fluidized bed bioreactors, single-use or non-single-use equipment, vertical or horizontal, high density cell culture systems, multilayer flasks, petri dishes, plastic discs among other methods and materials known in the state of the art. In a preferred context, these surfaces are made of plastic specially treated for cell adhesion, in T 225 cm² flasks.

The SARS-CoV-2 virus used in the experiments may be a human or animal isolate, primary isolation product or clone or subclone separation procedure, or it may be partially or totally reconstructed by engineering techniques genetics, producing more productive variants or attenuated viruses. In a preferred context, the viral isolate is SARS-CoV-2/SP02/human/2020/BRA (GeneBank MT126808.1), kindly provided by Prof. Edison Luis Durigon, from the Institute of Biomedical Sciences at USP, whomwith the seed and work banks were prepared.

For viral production, an amount of virus at a defined multiplicity of infection MOI is inoculated into the cultured cells. You can use several different MOIs, such as 0.00001; 0.0001; 0.001; 0.01; 0.1; 1; 10; 100 and intermediaries thereof. In a preferred context, the MOI used is 0.1. Said virus is added to cells with or without changing the present culture medium. In a preferred context, the culture medium is drained and the virus is inoculated in a volume of 3 ml per flask. After virus inoculation, the set of cells is incubated for a period of 1 to 120 h. In a preferred context, the cells are incubated with the virus for 1 h, after which the volume of culture medium is made up to the final volume required in the culture flask, preferably this volume is 100 ml. The flasks are then incubated for 2 to 120 h for virus production at a temperature between 24°C and 37°C. Virus production is preferably carried out within 48h with incubation at 37°C, as seen in Figure 1.

After the necessary incubation time, the virus is separated from the cells in the collection step in which the contents of multiple vials are gathered into a vial when necessary, using prior art techniques such as inversion, aspiration or pumping. The collection step is not necessary in virus production that occurs in a single vial.

After the collection step, the material is preferably sent for purification. In a preferred context, the first purification step is the removal of cell debris and other microscopic particles by means of clarification. The clarification process can be carried out by conventional or continuous flow centrifugation; ultracentrifugation; removal aided by ionic charges or magnetism; sedimentation or precipitation; filtration. In the preferred context, the removal of particles is carried out by means of filtration.

In the filtration step for clarification or for sterilization or reduction of microorganisms in the material, different types of filters or filtration techniques can be used. Membranes composed of polyester, PVDF, cellulose or regenerated cellulose, among others, can be used in different porosities, including the possibility of using devices with different combined membrane porosities. These membranes can be positioned in holders for membranes, cartridges, capsules, or other items where it is possible to separate the side where the material to be removed will be retained from the filtered material. In a preferred context, filtration is carried out using a capsule with a polyethersulfone membrane and a porosity of 0.22 µm.

The viral material may be purified active or inactive for the removal of remains of culture medium, cellular proteins and non-viral genetic material. This purification may be carried out by various techniques such as ultracentrifugation, chromatography, filtration, tangential filtration, ultrafiltration, precipitation and other techniques typical of the purification of biotechnological products.

In a preferred context the viral sample is concentrated to 1/2 to 1/10 of the original volume before purification. In a preferred context, this concentration is carried out by tangential filtration technique in a 300 kDa membrane at 1/8 of the original volume. Concentration can also be carried out by membranes composed of polyester, PVDF, cellulose or regenerated cellulose, among others in different porosities, including the possibility of using devices with different combined membrane porosities.

After concentration or in a process combined with it, or from the volume of material collected or clarified without concentrating, the material may be subjected to a solvent exchange process. The new solvent will be composed of an aqueous solution with pH buffering. The buffer solution may be composed of several components, the most common being sodium and potassium salts, phosphates, citrates or carbonates, buffers such as TRIS, HEPES or other buffers regularly used in virus and viral protein purification. In a preferred context, diafiltration will be carried out on a 300 kDa membrane consisting of PES in three diavolumes for the exchange of the viral suspension solvent. The composition of the membrane can be varied, as explained above. The amount of diavolumes used can also be varied, and does not consist of a variation on the technique described herein.

Another purification step is the reduction of contaminants in the material through chromatography processes of various types, such as but not limited to ion exchange, molecular exclusion, affinity, immunoaffinity, isoelectric, reverse phase, etc.

In another context, the purification is carried out by ultracentrifugation in sucrose mattress and sucrose gradient. In another scope, the ultracentrifugation process can be carried out in the presence of other agents that form phases or gradients, such as cesium chloride and iodixanol. In a preferred context, ultracentrifugation is carried out first with the concentrated material being positioned in a sucrose gradient and in a zonal rotor and the process is carried out by ultracentrifugation (Zonal 1), followed by (Zonal 2). SARS-CoV-2 may be recovered from different phases of the gradient between different concentrations of separation reagent. Preferably the virus will be taken from fractions 55-28% (zonal 1) and 55-35% (zonal 2).

The product reached from the purification has proven stability of up to 07 days in a cold room (6°C ± 2°C). The product can be dialyzed in a buffer solution or diafiltered under conditions already described for changing the buffer and removing the gradient-forming reagent, or even the salts and compounds used for its elution from chromatographic columns. The product can be frozen between -20°C and -196°C to preserve the active pharmaceutical ingredient. Preferably the product will be frozen at -80°C. The final sterilization of the product can be carried out using the same conditions and membranes mentioned in the "clarification" item. Preferably the filtration will be carried out using 0.22 µm polyethersulfone membrane.

The final product will be obtained from the combination or not of purified and inactivated virus with adjuvants. Adjuvants may be composed of aluminum salts, squalene and bacterial protein derivatives, in aqueous solutions or emulsions, combined or not. In a preferred context, the adjuvant used is aluminum hydroxide.

The viral inactivation process, carried out right after the collection, clarification or purified product step may be carried out by adding chemical reagents or using gamma radiation. The chemical reagents used can be, but are not limited to Betapropiolactone, formaldehyde, ascorbic acid, lactic acid, etc... Various concentrations and incubation times can be used for inactivation.

Preferably the chemical inactivation will be carried out with betapropiolactone at an active concentration of 0.05% and incubation at 4°C for 24 hours, followed by incubation at 37°C for two hours.

The inactivation by gamma radiation will preferably be carried out by the application of 15 kGy (kilo Grays) for XXX minutes, both for purified virus samples and for virus samples contained in culture plates, test tubes, microtubes, glass flasks, polypropylene or other plastics. Virus samples may be inside or outside the cells, in buffer solutions or not, having or not been previously treated for inactivation by another methodology.

Figure 3 shows a detailed flowchart with all stages of the production process of an antigen, corresponding to the inactivated SARS-COV-2 virus.

### Inactivation check

Verification of inactivation is carried out by incubating the inactivated virus sample in cell culture. In one approach, incubation can be carried out from two to fourteen days in one passage or for the same period in more passages until the presence or absence of cytopathic effect or the presence of viral multiplication is verified. Preferably, the incubation will be carried out for three passages with incubations of three or four days each and the cells will be lysed for analysis of viral RNA content. Viral RNA can be verified through different techniques, preferably it will be quantified by real-time RT-PCR. Preferably, the quantified RNA is the viral subgenomic RNA, present only in situations where the virus is active. Quantification of subgenomic RNA in RNA samples extracted from cells is carried out by inoculating specific amounts of extracted total RNA for reverse transcription. Preferably 40 ng of total extracted RNA is used. After reverse transcription, oligonucleotide primers (primers) are used to carry out the amplification in the presence of probes or base-intercalating reagent. Primers are preferably sequences based on published scientific work (Wölfel *et al.* 2020). The inactivation verification is done by maintaining the amount of subgenomic RNA detected (residual), and if the amplification of larger amounts of material is observed at each passage of the viral sample in the cells, the material should be considered active.

The verification of active viral titer can be carried out by plate titration or using amplification in real-time thermocycler or equivalent. For plate titration, serial dilutions of the viral sample will be carried out, which will be used for the inoculation of cells in culture plates. Preferably these cells will be VERO cells described above. Culture plates will be incubated for 24 to 72 h to observe the cytopathic effect of virus samples. Preferably, plates containing virus serially diluted in base 10 and cells will be incubated for 72 h. The culture medium will be removed from the plates and the remaining cell mat will be observed directly or fixed and stained. The mat can be fixed with different reagents such as formaldehyde, methanol, acetone or acetic acid and stained with different dyes, preferably fixed with acetic acid and stained with Naphthol blue. The titration of infectious virus will be done by determining the infective dose in tissue culture 50% (TCID50), based on the method by Reed & Muench, 1938.

Real-time PCR titration can be carried out by detecting any gene segment of SARS-CoV-2. Preferably, it will be done following the protocol of the University of Charité (https://www.who.int/docs/default-source/coronaviruse/protocol-v2-1.pdf?sfvrsn=a9ef618c_2), adapted to absolute quantification based on a curve of quantification. The viral quantification curve can be obtained through the use of virus dilutions, infected cell lysate, DNA fragments, plasmid vector dilutions or synthetic or extracted viral RNA dilutions or its fragments. Preferably, the quantification curve will be carried out by diluting double-stranded DNA fragments. A correlation curve between the infecting virus titer and the number of copies of the amplified genetic material should be carried out at each dilution of the curve in order to define a conversion factor between the initial number of amplified copies and, therefore, the amplification cycle generated by the equipment, and the title of infectious virus.

The extraction of viral genetic material may be carried out from viral suspension or cell sample. Preferably viral RNA will be extracted from viral suspensions. For RNA extraction, column-based extraction reagents and extraction kits can be used.

The extracted RNA will undergo the reverse transcription process using random primers. Oligo-dTs or specific to generate cDNA suitable for amplification. Reverse transcription can be carried out as part of the same amplification cycle (one-step) or in a process prior to performing the polymerase chain reaction (PCR). PCR can be carried out using fluorescent probes specific to the amplified fragment or using a base-intercalating fluorescent reagent. Preferably, a base-intercalating type reagent will be used to allow the analysis of dissociation curves after PCR.

After verification of the recognition of antigens produced by specific anti-S antibodies (kindly provided by Dr. Edison Durigon) and by serum from patients positive for Covid 19 (identified by seroneutralization), the antigens are being used for immunization of mice for verification of immunogenicity. Then, they will be used to immunize horses and obtain heterologous sera for treatment.

Verification of inactivation was done by 2 methods:

### Method 1: Serial passage in Vero cell culture

100 µL of the sample are incubated with Vero cells (12-well plate) for 72 h and then 100 µL of this culture are transferred to a new plate for the same incubation period (repeated once more, totaling three passages).

Absence of cytopathic effect and TCID₅₀ in the final material indicate inactivation.

It has been determined that a TCID₅₀ is capable of generating at least 100 TCID50 at each incubation. Thus 1 TCID₅₀ / 100 µL is within an estimated detection limit for the test, as the titration assay is capable of detecting up to 102 TCID₅₀ / mL.

### Method 2: Subgenomic RNA amplification

Coronavirus has a "unique" subgenomic RNA from the intracellular stage of its cycle.

A DNA fragment was synthesized containing the protein E sequence (target prot Charit6) and region of the fragment corresponding to the subgenomic RNA (Figure 5).

Amplification of subgenomic RNA in a kinetic study of infection can identify whether there is active virus, as shown in table 1.

**Table 1 - Subgenomic RNA amplification in a kinetic study of infection**

| | | | | | Copies/ng (RNA SUBG) | |
|---|---|---|---|---|---|---|
| Number | Sample | Inactivator | Concentration/load | 1^{st} Pass | 2^{nd} Pass | 3^{rd} Pass |
| 403 | Vero Control (MOI 0.02) + | NA | | 5.42 E±07 | | |
| 447 | Vero Mock | NA | | 3.0±0.3 | | |
| 318; 423 | SARS lot 03/20 | Betapropyloctone | 0.025% | 92.3±8.7 | 24.8±14.0 | |
| 322; 431 | SARS lot 03/20 | Betapropyloctone | 0.05% | 225.4±39.2 | 35.1±3.6 | |
| 439 | SARS lot 03/20 | Formaldehyde | 0.10% | ND | 42.4±8.4 | |
| 327; 441 | SARS lot 01/20 Radiated 227 | Gamma radiation | 10kGy | ND | 16.6±1.1 | 6.1±5.6 |
| 328; 443 | SARS lot 01/20 Radiated 228 | Gamma radiation | 15kGy | ND | 14.1±2.4 | 9.2±3.9 |
| 328; 445 | SARS lot 01/20 Radiated 229 | Gamma radiation | 25kGy | ND | 17.0±4.1 | 4.1±2.0 |

Figure 6 shows the inactivation of serum 450, Serum 761 and human serum 861.

Irradiation (5-25kGreis) of human plasma does not alter overall coagulation tests.

### Blood collection

Human blood was obtained via venipuncture in a consensual way, from apparently healthy individuals who were not using medications such as contraceptives and antiinflammatory drugs for at least 10 days before collection (Plataforma Brasil (Brazil Plataform) - No. - 32443020.4.0000.5501) and collected in tubes containing 3.8% sodium citrate in the citrate:blood (1:9 v/v) ratio.

### Preparation of Platelet-Poor Plasma (PPP)

To obtain platelet-poor plasma (PPP), human blood was centrifuged at 2,500 g for 20 minutes in an Eppendorf brand centrifuge (model 5810R) at 25°C. The PPP obtained was fractionated into 1 mL aliquots that were stored at -20°C until use.

### Platelet-Poor Plasma Co-60 Source Irradiation

The Platelet-Poor Plasma was submitted or not (control) to irradiation with a Co-60 source at doses of 5 to 25 kGy (kilograys) in the multipurpose irradiator of the Radiation Technology Center (CETER) of the Energy Research Institute and Nuclear (IPEN) - CNEN/São Paulo.

### Global Coagulation Tests

After irradiation or not (control), the PPP was submitted to global coagulation tests, Prothrombin Time (PT) and Partially Activated Thromboplastin Time (PaTT), to assess the percentage of maintenance of the activity of the coagulation enzymes blood. The tests were carried out as described below:
a) Determination of prothrombin time (PT): To determine the prothrombin time, the reagent (Hyphen^{®}) reconstituted at the time of use was used, according to the manufacturer's instructions. In a semi-automatic coagulometer (Diagnostica Stago - França), the platelet-poor plasma (100 µL) exposed to different doses of ionizing radiation (5 to 25 kGy) was incubated for 2 minutes at 37°C. After incubation, 100 µL of thromboplastin was added and the timer started. These experiments were carried out in the Development and Innovation Laboratory and the determinations were carried out in triplicate.
b) Determination of Partially Activated Thromboplastin Time (PaTT): The determination of partially activated thromboplastin time was carried out in a semi-automatic coagulometer (Diagnostica Stago - France). Platelet-poor plasma (100 µL) exposed to different doses of ionizing radiation (5 to 25 kGy) was incubated at 37°C for 3 minutes with 100 µL of reagent (cephalin and ellagic acid - Hyphen^{®}). After incubation, 100 µL of CaCl₂ was added and the timer started. These experiments were carried out in the Development and Innovation Laboratory and the determinations were carried out in triplicate.

### Results

Figure 7 shows the result of the prothrombin time, where the platelet-poor plasma (100 µL) was exposed to different doses of ionizing radiation (5 to 25 kGy). After incubation, 100 µl of thromboplastin was added and the clotting time measured. Columns represent the average of triplicates ± SD. (- %) = percentage of coagulation enzyme activity.

Figure 8 shows the partially activated thromboplastin time, where the platelet-poor plasma (100 µL) was exposed to different doses of ionizing radiation (5 to 25 kGy). After incubating the PPP with cephalin and ellagic acid, 100 µL of CaCl₂ were added. Columns represent the average of triplicates ± SD. (- %) = percentage of coagulation enzyme activity.

The inactivation by GAMMA IRRADIATION (10-25 kGreis) of culture media exposed to the virus does not change the protein profile of the media, and can be used in laboratory tests to be developed in clinical analysis laboratories, research or production of biotechnological products.

Viral inactivation by radiation of cell culture supernatants for use in clinical analysis laboratories, research or production laboratories of biotechnological products guarantees the safe use of samples in Laboratories that do not have qualification of biological safety levels (for example NB2, NB3), to work with infectious agents such as viruses, such as SARCS COV-2. The technique used for inactivation was shown not to affect the quantification of proteins present in cell culture supernatants, making its measurement possible.

### EXAMPLES

### Example 1: Macrophage supernatants derived from the monocytic lineage THP-1 were irradiated with 10, 15 and 25 KGreis and inflammatory cytokines were quantified.

### Methodology

*Obtaining macrophages*: THP-1 monocytes were differentiated into macrophages using the protocol described and characterized by Lund *et al.* (2016). Briefly, the cells (2×106) were incubated for 48h in 3 ml of RPMI medium containing 25nM of PMA. After this period, the macrophages were kept at rest for 24 hours in a PMA-free medium.

*Treatment*: Macrophages, after the differentiation protocol, were treated for 24 hours with: LPS (1 pg/mL) for induction of expression and release of inflammatory cytokines.

*Analyzes*: Supernatants from macrophages treated with LPS were collected, subjected to irradiation and the concentrations of IL-4, IL-6, IL-8, IL-10, TNFα and IL-1β released were evaluated, both in irradiated and non-irradiated samples, using Multiplex kits (Merck) in the Luminex equipment, following the manufacturer's protocol.

### Results

Supernatants collected from macrophages derived from the monocytic lineage THP-1, stimulated with LPS, non-irradiated and irradiated with doses of 10kGy, 15kGy and 25kGy had the inflammatory cytokines IL-6, IL-8, IL-1β, IL-4, quantified IL-10 and TNFα (Figure 9). The data showed that none of the tested irradiation doses affected the quantification of inflammatory cytokines when compared to the quantification of non-irradiated supernatants.

Figure 9 shows the quantification of inflammatory cytokines using Multiplex kits (Merck) in the Luminex equipment in THP-1 cell supernatants after irradiation with doses of 10kGy, 15kGy and 25kGy. The cytokines IL-6, Il-8, IL-1β, IL-4, IL-10 and TNFα were measured.

### OTHER EMBODIMENT OF THE PRESENT INVENTION.

### Production of purified and inactivated SARS-CoV-2

### SARS-CoV-2 Bank

SARS-CoV-2/SP02/2020HIAE (GeneBank MT126808.1. B.1.1.28) is a clinical isolate of a patient admitted to Hospital Israelista Albert Einstein (São Paulo, Brazil). The clinical material was tested for SARS-CoV-2, according to Corman *et al.,* (2020), and for 15 viral agents by real-time PCR (Influenza A and B, endemic coronavirus - CoV-NL63, -229E, -HKU1 and -OC43, Enterovirus, Parainfluenzavirus 1, 2, 3 and 4; Human Metapneumovirus, Rhinovirus, Respiratory Syncytial Virus and Adenovirus) (Araujo *et al.,* 2020), which were all negative. The virus was isolated in VERO E6 cells and the characterization of the strain is described by Araujo *et al.,* (2020). Working virus seed stock (WVSS) was prepared by infecting VERO CCL-81.4 ECAAC General Collection 88020401, adapted to VP serum-free medium (ThermoFisher, MA, USA) at the Butantan Institute with an MOI (multiplicity of infection) of 0. 1 for 48 h, 37°C and 5% CO₂. The virus produced was sterilized by filtration (0.22 pm), formulated with SPG (7.462 g / L sucrose, 0.0517 g / L KH₂PO₄, 0.1643 g / L K₂HPO₄.3 H₂O and 0.0907 g / L potassium glutamate) and frozen at -80°C. The final WVSS titer was 9.79E + 06 TCID50 / mL. All manipulations of the active virus were carried out in a BSL3 laboratory.

### Virus titration - Determination of tissue culture infectious dose 50% (TCID50)

Virus samples were taken from the culture or purification steps and immediately frozen at -80°C before analysis. After defrosting, the samples were serially diluted ten times (10⁻¹ - 10⁻¹²) in Dulbecco's modified Eagle's medium (DMEM) with 2.5% fetal bovine serum (FBS) and inoculated in six replicates in glass plates of 96 wells seeded with VERO ATCC CCL-81.4 cells (2 × 10⁴ cells/well). After incubation at 37°C with 5% CO₂ for 72 h, the plates were microscopically inspected for cytopathic effects (CPE) caused by SARS-CoV-2. To confirm the results, the monolayers were fixed and stained with Naphthol Blue Black (Sigma-Aldrich) solution (0.1% naphthol blue w/w with 5.4% acetic acid and 0.7% acetate sodium) and analyzed. Viral titer was calculated using the Spearman & Kärber algorithm as described by Hierholzer & Killington (1996) and was expressed as TCID50/mL.

### Production and inactivation of antigens

For antigen production, Vero cells (ATCC CCL-81.4) were seeded in 225 cm² T flasks (10⁷ cells/flask) and cultured for 48h before infection. SARS-CoV-2 / SP02 / 2020HIAE was inoculated at an MOI of 0.1 in 5 mL of culture medium and allowed to adsorb for one hour at 37°C. Serum-free medium was added to the cultures until reaching the volume of 100 mL. Cultures were incubated at 37°C, 5% CO₂, for 48h. Before harvesting, the flasks were microscopically inspected for cytopathic effects and absence of contamination, and then the culture medium was drained into a new flask. Three batches of active SARS-CoV-2 were clarified by 0.22 µm filtration (Opticap XL2 Durapore, MerckMillipore) and pooled (6 L) for the following purification process. The clarified material was concentrated by tangential flow filtration (TFF) using a Minipellicon system with an Ultracell 300 kDa membrane (MerckMillipore). The concentrate (2 L) was subjected to two rounds of ultracentrifugation (XPN 90, BeckmanCoulter) in sucrose gradients (650 - 34%) using a Ti15 zonal rotor for 3 h at 4°C and 25 krpm. Fractions containing purified virus were pooled after each ultracentrifugation procedure. The final purified material was diluted with 1X PBS to a sucrose concentration of 20%, filter sterilized (0.22 µm) and frozen at -80°C.

Frozen aliquots of purified SARS-CoV-2 were subjected to virus inactivation by gamma irradiation at the Nuclear Research Institute (IPEN, São Paulo, Brazil). In this sense, the viral suspensions were submitted to the inactivation process by gamma radiation (Gammacell 220 Irradiation unit, Nordion, Canada). The radiation dose to inactivate 1 log virus titer was initially determined (D10). First, seven virus samples were irradiated with different doses of gamma radiation (0.1, 2.5, 5.5, 10, 15 kGy). Then, another experiment was carried out to confirm D10 with seven radiation doses (0, 1, 2, 3, 4, 5, 6 kGy). Virus titer was quantified by determining TCID50/mL.

### Virus Inactivation Test

The effectiveness of the SARS-CoV-2 inactivation process was evaluated by detection of cytopathic effect and kinetic analysis of subgenomic viral RNA by RT-qPCR in VERO CCL 81.4. after inoculation of the SARS-CoV-2 antigen. Briefly, 0.1 mL aliquots of the inactivated virus were incubated with 1E + 05 VERO CCL - 81.4 cells for 1 h at 37 °C, followed by the addition of 1.4 mL of DMEM + 10% FBS culture medium. The plates were incubated for 72 hours and, in the absence of cytopathic effect, a 0.1 ml aliquot of the supernatant was transferred to a new VERO CCL 81.4 cell culture for the second passage. The procedure was repeated at least one more time. For kinetic analysis of subgenomic viral RNA, 1 mL of inactivated virus was inoculated in 0.1 mL aliquots in ten wells of a 12-well plate containing VERO cells for 72 h. The supernatant from all wells was pooled and 0.1 ml aliquots were placed in a new well of a 12-well plate for the second passage in VERO cells.

For the analysis of increasing amounts of subgenomic RNA during incubation, indicative of virus replication, samples were collected after 1 h, 24 h and 48 h of incubation by monolayer cell lysis with PureLink^{™} lysis buffer RNA Mini Kit (ThermoFisher), following the manufacturer's instructions. RNA was extracted and 100 ng of total RNA was reverse transcribed followed by qPCR using High-Capacity cDNA Reverse Transcription and Fast SYBR^{™} Green Master Mix kits, respectively (ThermoFisher). Quantitative PCR was carried out using serial dilutions of a synthetic sequence corresponding to the amplification fragment as standard curve (GeneBlocks, IDT). Primers were synthesized based on published methodology (Wölfel et *al.,* 2020) .

### Antigen characterization: purity, identity and quality

The purity of the inactivated virus samples was evaluated by SDS-PAGE using 4-15% CriterionTM TGXTM Precast Midi Protein gel, under non-reducing conditions (Bio-Rad). Electrophoresis was carried out for 1 h at 100 V and the gel was stained using Pierce^{™} Silver Stain Kit (Thermo Fisher Scientific). In addition, the amount of residual VERO cellular protein was quantified by the VERO Cell Host Cell Protein Immunoenzyme Assay (Cygnus Technology) as recommended by the manufacturer. The purity and identity of the antigen were also carried out by means of mass spectrometry analysis.

The endotoxin content in the purified and inactivated virus sample was quantified using the PYROGENTTM Plus Gel Clot LAL assay (Lonza, USA), according to the manufacturers' recommendations.

The quality of the antigen was analyzed based on the preservation of the antigen after the inactivation process by Western blotting. For this analysis, 400 µL of purified and inactivated antigen were subjected to SDS-PAGE as described above, and proteins transferred to nitrocellulose membrane for 1 h at a current of 90 V - 150 mA. The membrane was blocked with the blocking solution for 3 h at room temperature, under agitation. Then, the membrane was washed with phosphate buffer plus 0.05% Tween 20 (3 × 5 min), followed by incubation with rabbit monoclonal antibody [CR3022] to SARS-CoV-2 Spike Glycoprotein (Abcam - Ab273074) and rabbit polyclonal antibody to SARS Protein -CoV-2 N, kindly provided by Dr. Roxane Piazza (Instituto Butantan, Brazil), diluted in sample diluent, for 1 h. Peroxidase (KPL) labeled goat anti-rabbit IgG antibody was used for labeling and the membrane was developed with chemiluminescent substrate Super Signal West Peak (ThermoFisher). The images were acquired with a Uvitec Alliance 2.7 equipment.

### Inactivated virus immunogenicity analysis

Female BALB/c mice (18 - 22 g) were obtained from the Center for Animal Breeding at the Butantan Institute. The use of mice in the present study was approved by the Institutional Animal Care and Use Committee of the Butantan Institute (approval protocol n° 9165080620). Animals (n = 5) were immunized subcutaneously with purified and inactivated SARS-CoV-2 (corresponding to 1.74 × 104 TCID50 / animal) mixed with Al(OH) 3 (0.25 mg / animal) to achieve a final volume of 200 µl. These animals received three booster doses (1.74 × 104 TCID50 / animal) at 7-day intervals. Control animals (n = 5) were inoculated with sterile saline solution with Al(OH) 3 (0.25 mg/animal). Bleeds were carried out 21 and 35 days after priming for serum collection and samples were stored at -80°C until use. Serum samples were further analyzed by Elisa (as described in section 2.2.8.1) or in the cytopathic effect-based virus neutralization test (CPE-VNT). For CPE-VNT, sera were previously inactivated at 56°C for 45 min.

### Anti-SARS-CoV-2 Equine Immunoglobulin Production

### Animals

The procedures involving the horses were approved by the Institutional Committee for the Use and Care of Animals of the Butantan Institute (approval protocol n° 6016030720). Ten healthy horses, not previously immunized, were selected and trained to be manageable for the work. Clinical status and behavior were assessed and those that were adequate had blood samples collected for laboratory tests, including complete blood count (red blood cells, leukocytes, platelets, hemoglobin, hematocrit), basic metabolic panel (calcium, glucose, sodium, potassium , blood urea nitrogen, creatinine), complete metabolic panel (total protein, albumin, gammaglobulin, alkaline phosphatase, alanine aminotransferase, aspartate aminotransferase, bilirubin), lipid panel (high density lipoprotein, low density lipoprotein), creatine phosphokinase, tests coagulation (prothrombin time test and activated thromboplastin time test). Blood samples were collected from the jugular vein and analyzed in a veterinary clinical laboratory.

### Immunization

Horses (n = 10) were injected four times with viral inactivated SARS-CoV-2 suspension (corresponding to 4.5 × 105 TCID50/animal/dose). Subcutaneously, at an interval of one week, MontanideTM ISA 50V oil-in-water adjuvant (Seppic; Shanghai, China) was included in the first and third doses to enhance the antibody response. Serum samples were collected before and during immunization and stored at -20°C until use. Hematological and biochemical parameters and antibody titers against SARS-CoV-2 proteins (by ELISA) were monitored before and after the immunization schedule.

### Plasma collection

Plasma was collected by automated plasmapheresis, using the Cobe Spectra^{®} system (Terumo BCT) for therapeutic procedures of apheresis. Briefly, a double-lumen catheter was inserted into the jugular vein. One way was to draw whole blood from the horse, which was added to the ACD anticoagulant. Blood components were separated based on the specific gravity of the cells by continuous flow centrifugal technology. Plasma was collected in a temporary bag and transferred to a custom one. The other form of the catheter allowed the return of uncollected components to the animal with Ringer's lactate solution. The plasmapheresis procedure was carried out three times (days 28, 35 and 49 after the first) according to the scheme presented below:

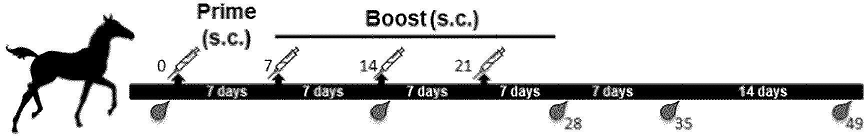

Each plasmapheresis procedure generated a batch of hyperimmune plasma, therefore, three batches were obtained sequentially. Plasma samples were analyzed for bioburden and antibody titres tests.

### Immunoglobulin purification

The initial plasma pool was subjected to fractionation by precipitation with ammonium sulfate. The precipitate was centrifuged and the liquid fraction was discharged. The product was then solubilized and pH adjusted for enzymatic digestion by pepsin to hydrolyze non-IgG proteins and cleave the molecule into F(ab')2 fragments, removing the Fc fragment. A second precipitation was carried out by adding ammonium sulphate and the pH was adjusted by adding caprylic acid and stirring. Then, thermocoagulation at 56°C separated the nonspecific thermolabile proteins and the precipitate was eliminated. The supernatant was subjected to diafiltration, ion exchange chromatography and concentrated by tangential ultrafiltration. Phenol was added as a preservative and the chloride concentration was adjusted. The F(ab')2 solution was then subjected to 0.22 µm filtration and kept in disposable plastic bags. All production steps were carried out under GMP conditions.

### End Product Protein Profile (Batch 00001, 00002, 00003)

The protein profile of the final product batches was analyzed using SDS-PAGE (12.5%) under reducing conditions. To assess the existence of any proteolytic products, samples from each batch were incubated at 37°C for 30 min, 1 h, 2 h or 3 h and subjected to SDS-PAGE. Gels were stained using Coomassie Blue stain (40% methanol, 10% acetic acid and 0.25% Coomassie blue) and destined using 400 methanol with 10% acetic acid.

### Protein identification through LC-MS / MS analysis (liquid chromatography and tandem mass spectrometry)

Samples of the purified inactivated virus (8911/20 batch) and the final anti-SARS-CoV-2 serum product (Batch 00001, 00002, 00003) were submitted to the FASP protocol (Wiśniewski *et al.,* 2009) using units of 10 kDa filter (Merck-Millipore). The tryptic peptides were desalted using homemade stage tips, the eluted peptides were vacuum dried using a centrifugal concentrator (Eppendorf). The samples were dissolved in 20 µl of aqueous buffer containing 0.1% formic acid and two microliters of each sample were injected onto an Acclaim PepMap100 C18 trap column (Thermo Scientific) 3 µm particle size, 100 µm particle size pore, 75 um internal diameter, 20 mm column and analyzed by an Orbitrap Q-Exactive plus mass spectrometer (Thermo Scientific) coupled to an Easy nanoLC 1200 (Thermo Scientific) with a flow rate of 200 nl / min , buffer A contained 0.1% (v/v) formic acid and buffer B 0.1% (v/v) formic acid in 80% acetonitrile. Peptides were applied with an increasing gradient of acetonitrile over time (0-90% (v/v) acetonitrile in 30 min) to separate the peptides on the analytical column Acclaim PepMap Column (Thermo Scientific) 2 µm particle size, 100 Å pore size, length 150 mm, inner diameter 50 µm. The MS was operated in data-dependent mode with one of the 7 main methods at a mass-to-charge ratio of 300-2000. The spray voltage was set at 2.4 kV and the mass spectrometer was operated in the positive data-dependent mode, in which a full MS scan was acquired in the m / z range of 300-1500 followed by the MS / MS acquisition using high energy collisional dissociation (HCD) of the seven most intense ions from the MS scan using a 2.0 m/z isolation window.

### LC-MS / MS data analysis

The obtained MS and MS/MS spectra were analyzed using PEAKS STUDIO version X and the searches were carried out against specific database built. For the analysis of antigen data, the reference bank used included all SARS-CoV-2 sequences and revised Chlorocebus aethiops sequences available from Uniprot (total of 162 sequences). For data analysis of the three batches of anti-SARS-CoV-2, the reference bank was constructed using all anti-SARS-CoV-2 sequences, available from GeneBank and revised Equus caballus sequences from Uniprot (total of 2,190 sequences). Both reference databases were concatenated with common contaminants for mass spectrometry experiments (116 sequences) and decoy sequences were used for false discovery rate control. The search engine was configured to detect semi-tryptic peptides at an FDR of 0.01. Oxidation of methionine and acetylation of the N-terminus of the protein were defined as variable modifications and carbamidomethylation of cysteine was defined as a fixed modification. Features identified as contaminants and proteins without unique peptides were filtered out. Spectral counts were used to estimate the proportion of proteins in each sample.

### Anti-SARS-CoV-2 serum potency analysis

### ELISA for detection of anti-SARS-CoV-2 IgG

To assess the serum antibody titers of the immunized mice and horses, ELISA plates (Costar^{®}) were coated with 100 µL of purified and inactivated SARS-CoV-2 (4.2 × 104 virus / mL) and incubated during the overnight at 4°C. Plates were then blocked with 5% BSA in 1X PBS for 2 h at 37°C and incubated with increasing dilutions of non-immune or experimental serum samples for 1 h at room temperature. After incubation, plates were washed with PBS-Tween 20 0.05% and incubated with anti-mouse IgG-HRPO (1:2000) or anti-horse IgG-HRPO (1:5000) in BSA / PBS 0.1%, then the plates were washed and the reactions developed with TMB substrate (BD OptEIA^{™}) according to the manufacturer's instructions. Absorbances were recorded in an ELISA reader (BioTek Elx800 spectrophotometer) at 450 nm. The titer was established as the highest serum dilution at which the measured absorbance was twice as high as that determined for normal mouse serum (control group) or for pre-immune horse plasma.

### Neutralization virus test based on Cytopathic effect (CPE-VNT)

To assess the serum antibody titers of immunized mice and horses, CPE-VNT was carried out using the wild-type variant B.1.1.28 SARS-CoV-2 (SARS-CoV-2/human/BRA /SP02/2020 (MT126808.1) The 3 batches of the final product (anti-SARS-CoV-2 equine serum) were also tested against the variant P.1./Gamma SARS-CoV-2 (strain IMT 87201) and variant P.2./Zeta (hCoV-19/Brazil/RS-00601/2020 - LMM-EPI_ISL_779155, kindly provided by Dr. Fernando Spilki - Universidade Feevale, RS, BR). The assays were carried out in 96-well seeded plates 24 to 28 h before with 2 × 10⁴ cells/well of VERO cells (ATCC CCL-81.4.) A serial dilution of each serum (1:20 to 1:40960) in DMEM with 2.5% FBS was carried out and then 100 TCID50 of virus (v/v). The virus/serum mixture was incubated at 37°C for 1 h to allow virus neutralization. Then the mixture was transferred to the confluent VERO cell monolayer and incubated for 72 h at 37°C, under 5% CO₂. After the incubation period, the plates were again microscopically inspected for CPE caused by SARS-CoV-2 and subsequently stained with 0.2% Naphthol Blue Black solution and analyzed to confirm the titer. The virus neutralizing titer, referred to as VNT100, is described as the highest serum dilution that neutralized virus growth. In each assay, one positive serum and one negative serum were included as controls.

### cPassTM ELISA Kit

The cPassTM kit for detection of neutralizing antibodies to SARS-CoV-2 (GenScript, Cat.: L00847) was used according to the protocol provided by the manufacturer, for a direct qualitative detection of total neutralizing antibodies to SARS-CoV-2 in the antiserum. Initially, samples from the three lots of equine anti-SARS-CoV-2 were diluted 1:100, 1:1000 and 1:2500 with PBS buffer pH 7.4. The neutralizing serum plate was blocked with 200 pL/well of PBS + 1% BSA and then incubated at 37 ± 2°C for 1 to 2 h. Samples and controls were added to the wells and incubated at 37 ± 2°C for 30 minutes with RBD-HRP solution. The same samples and controls were then transferred from the serum neutralization plate to the capture plate (ELISA), incubated at 37 ± 2°C for 15 min and washed four times with a wash solution provided 1x (1:20 dilution with ddH2O, 300 µl per well). After the washing step, the TMB substrate solution was added (100 µL per well) and incubated at room temperature (25 ± 3°C) for 20 min, protected from light. Finally, the stop solution provided was added (50 µL per well) to stop the HRP and TMB reaction. The absorbance of the samples was immediately measured at 450 nm.

### Elecsys^{®}Anti-SARS-CoV-2 S

The three batches of anti-SARS-CoV-2 equine serum were diluted in sterile pyrogenic saline solution, at a ratio 2, from 1/20 to 1/20480. The samples were transferred to vacutainer-type collection tubes, compatible with Cobas and Roche equipment. Analyzes were carried out in an automated fashion, using the Elecsys^{®}Anti-SARS-CoV-2 kit (Roche, xx), for the quantitative determination of antibodies (including IgG) against the RBD domain of the SARS-CoV-2 Spike protein. Results were expressed in U/mL based on the Kit's standard monoclonal antibody curve, with 1 nM of the anti-RBD MoAb corresponding to 20 U/mL.

### Preclinical toxicology and safety studies

The protocols used in this study were approved by the Institutional Animal Care and Use Committee of the Butantan Institute (approval of protocol No. 9165080620). BABL/c male and female mice, weighing 20 to 25 g, were obtained from the Animal Breeding Center of the Butantan Institute. The room temperature during the study was maintained between 18 and 22°C, humidity of 50 and 65% and lighting on a 12 h light and 12 h dark cycle. Each cage was supplied with commercial food CR1-Nuvital Nuvilab^{®} and *ad libitum* water. Male and female New Zealand rabbits (twenty weeks old) were obtained from Granja-RG (Suzano, São Paulo Brazil) and kept in ventilated racks, under controlled temperature and humidity and 12 h / 12 h light/dark cycle with *ad libitum* access to food and water.

The safety studies were prepared following the guidelines of the Brazilian Regulatory Agency (ANVISA, 2013). All animal experiments followed the ARRIVE guidelines and were carried out in accordance with the UK Animals Act 1986 and associated rules. Briefly, for single-dose acute toxicity studies, the threshold dose was 200 µL for mice and 1.5 mL for rabbit. The animals were observed for 14 days (mice) or 90 days (rabbit) after administration of anti-SARS-CoV-2 serum. Groups of rats were divided into four groups of 20 animals each (10 males and 10 males) homogenized on the basis of weight and i.p. injected with aprirogenic saline solution (control) or with different doses of anti-SARS-CoV-2, i.e., 33,635 U / RBD / g. 3.365 U / RBR / ge 0.3365U / RBD / g, as determined by serum potency analysis (section 2.2.8.4). Two groups of 12 rabbits (6 males and 6 females), homogenized on the basis of weight, were i.v. injected with aprirogenic saline solution or 8.268 U/RBD anti-SARS-CoV-2 serum.

### Clinical evaluation of mice

The body mass and water consumption were evaluated throughout the experiment. Hippocratic screening was carried out by means of individual observations of the animals in the periods of 15 min, 30 min, 1, 2, 4 h after injection and, thereafter, every 24 h for 14 and 90 days after treatment. The state of consciousness and general disposition, motor coordination, muscle tone, reflexes and autonomic nervous system activity were evaluated. Blood collection from animals for hematological and biochemical analyzes was carried out in deeply anesthetized animals (10 mg/kg xylazine hydrochloride and 100 mg/kg ketamine hydrochloride). Hematological and biochemical parameters were evaluated using specific kits according to the manufacturers. All measurements were carried out using a ProCyte Dx Hematology Analyzer and Catalyst One Chemistry Analyzer (IDEXX Distribution, Wisconsin US).

### Histopathological analysis and relative organ mass

Animals under deep anesthesia were sacrificed, following a detailed external examination (color and integrity of skin and hair, changes in all tissues and the shape of the abdomen) and internal. Organs (kidney, spleen, liver, heart, lung, lymph nodes, Peyer's patches, testes, ovaries, skin and brain) from each animal were removed and weighed. The relative mass of the organs of each animal was calculated by dividing the weight of each organ (g) by the body weight of each animal on the day of collection and multiplying the result by 100.

### Challenge test on golden Syrian hamsters

### Study design

Seventy-two conventional golden Syrian hamsters (Mesocricetus auratus) (36 males and 36 females, 115-148 g, 10-11 weeks old) were obtained from the Animal Breeding Center of the Butantan Institute. Hamsters were individually housed and divided into four groups of 18 animals each (9 males and 9 males) homogenized on the basis of weight. On day zero, the animals were anesthetized with ketamine and xylazine, and two groups (G1 and G2) were inoculated intranasally with 50 µL of 1 × 10⁵ TCID50 of SARS-CoV-2 (SARS-CoV-2 / SP02 / 2020HIAE, GeneBank MT12680.1, 4^{th} passage in VERO cells) in DMEM with 2% FBS, while the two remaining groups (G3 and G4) were inoculated with 50 µL of DMEM 2% FBS.

On day 2 p.i. (post-infection), hamsters from G1 and G3 were treated with 500 µL of equine hyperimmune serum (7.2 mg / animal), while hamsters from G2 and G4 received 500 µL of sterile, non-pyrogenic saline solution, both intraperitoneally. On days 3, 5 and 7 p.i., subgroups of 6 animals (3 males and 3 females) from each group were sacrificed with overdoses of ketamine and xylazine and necropsied. Lung fragments were collected in lysis buffer (Mag MaxCore Thermo Fisher Scientific kit) (for RNA extraction), in viral transport medium [VTM - DMEM + 2% FBS + antibiotics / antifungals (Vitrocell - penicillin 10,000U, streptomycin 10 mg, 25 µg amphotericin B / mL] (for virus titration) and in 10% formalin (for histopathological analysis). All five lobes of the lungs were represented in each sample for RNA extraction and virus isolation, and analyzed separately for histopathology. Whole lungs and all tissue fragments were weighed. Samples collected for virus isolation were snap frozen in liquid nitrogen and stored at -80°C until processing. Animals were also bled and serum analyzed for the presence of equine antibodies by ELISA.

The procedures involving hamsters were approved by the Institutional Committee for the Use and Care of Animals of the Butantan Institute (approval protocol No. 9165080620) and the Institute of Biomedical Sciences of the University of São Paulo (approval protocol No. 6103261120). The experiments were carried out in a biosafety level 3 animal facility.

### Quantification of viral load of tissue samples

Quantification of viral load was carried out by determining TCID50 / g tissue (using samples in VTM) and determining viral RNA copy number / β actin RNA copies (using samples in lysis buffer) . Briefly, all nasal conchae, trachea and lung samples were thawed and ruptured using 2 mm glass beads in TissueLyser II equipment (Qiagen) at 30 Hz for 2 minutes twice, followed by centrifugation at 13,000 rpm (Eppendorf - Centrifuge 5804R) for 30 seconds. Supernatants were used to quantify the viral load. The determination of TCID50 was done as described above.

Total nucleic acid was extracted using the Magmax Core Kit in the MagMAX Express Magnetic Particle Processor (ThermoFischer Scientific) following the manufacturer's instructions. Detection of SARS-CoV-2 RNA was carried out based on a previously described protocol (Corman *et al.,* 2020) using a one-step quantitative real-time PCR (qRT-PCR) assay kit (AgPath-ID ^{™} One-Step) RT-PCR Reagents - Applied Biosystems Inc., Waltham, USA) in an ABI 7500 SDS real-time PCR machine (Applied Biosystems, Weiterstadt, Germany). The reaction was carried out as a duplex using SARS-CoV-2 specific primers and β actin primers (ActB Rv 5'CAC CAT CAC CAG AGT CCA TCA C 3', ActB F CTG AAC CCC AAA GCC AAC; ActB_P-HEX TGT CCC TGT ATG CCT CTG GTC GTA ZEN / IOWA BLACK) as a maintenance gene. The number of RNA copies/mL was quantified based on a standard curve obtained by serially diluting a synthetic dsDNA sequence (GeneBlocks, IDT) corresponding to the amplification fragment of the target gene.

### Capture-ELISA for detection of horse antibodies in hamster serum samples

Capture-ELISA assays were carried out to identify horse antibodies in hamster serum samples. Briefly, ELISA plates were coated with 100 µl of rabbit anti-horse IgG (500 ng/ml) (Sigma) overnight at 4°C. Plates were washed with 200 µl PBS and blocked with 200 µL of 5% BSA in PBS for 2 h at 37°C. Plates were then washed three times with 200 µL of PBS, and the standard curve previously prepared (anti-SARS-CoV-2 horse F (ab') 2 serially diluted in PBS, from 262 µg / mL to 0.13 µg / mL) and serum samples (diluted 1/10 in PBS) were added in triplicate. After 1 h of incubation at 37°C, plates were washed three times with PBS / 0.05% Tween 20 and incubated with peroxidase-conjugated anti-horse IgG (diluted 1/5000 in PBS / 0.05% Tween 20 / 0.1% BSA (Zymed Technologies)), for 1 h at 37°C. Plates were washed three times with PBS / 0.05% Tween 20 and reactions performed for 15 min with TMB substrate (BD OptEIA^{™}), according to the manufacturer's instructions. Absorbances were recorded in an ELISA reader (BioTek Elx800 spectrophotometer) at 450 nm. The standard curve was linearized by log-log transformation and then submitted to linear regression. The concentration of horse F (ab') 2 in serum samples was estimated by interpolating the samples to the standard curve.

### Histopathological examination

After euthanasia, the lungs were extracted from the hamsters and fixed in 10% formalin for 24 hours. Lung samples were then subjected to routine histological fixation and stained with hematoxylin and eosin (HE). Histopathological findings for SARS-CoV-2 related changes were evaluated based on the literature (Roberts *et al.,* 2005; Gruber *et al.,* 2020; Geramizadeh *et al.,* 2020).

Tissue samples were examined under a light microscope for the presence of cellular/tissue changes, as described in detail in Table S1 (Supplementary Material), and a histopathological score was determined, being very mild (I), mild (II), moderate (III) and severe (IV). Lung sections were evaluated blindly by certified veterinary pathologists. The metric allowed us to generate a numerical score for the degree of bronchitis, edema, and pneumonia to determine the therapeutic effectiveness of treatments (Kreye *et al.,* 2020). For this purpose, five anatomical structures of the lungs were analyzed per animal, identified as: right cranial lobe; right middle lobe; right tail lobe; accessory lobe and left lung.

### Analysis of leukocyte infiltration in lung tissue

In order to better analyze the inflammatory process, the number of neutrophils, monocytes and lymphocytes present in the lung tissue of hamsters was determined. For this purpose, five fields per animal/slide were captured in a digital camera (DS-Filc Digital Camera from Nikon) using the microscope (Nikon Eclipse Ti-S, Melville, NY). The used images were stained with eosin and hematoxylin. For the leukocyte count, the ImageJ software and the Color Deconvolution 2 plugin were used to visualize the nuclei separated from the cytoplasm (Fig. S1 - Supplementary Material), which implements the separation of stains by the method of Ruifrok & Johnston (2001). For cell count, the Cell Counter plugin was used. This analysis allowed for the differential count between segmented and mononuclear nuclei.

### Immunofluorescence

Thin sections of lung tissue were subjected to deparaffinization, rehydration and antigen retrieval. Endogenous peroxidases were then blocked, followed by tissue permeabilization using 0.4% Triton X-100 in PBS. Nonspecific binding was blocked and thin sections were washed twice with PBS-Tween and treated with anti-SARS-CoV-2 peak glycoprotein primary antibodies (abcam, ab272504, 1:100) for one hour at room temperature. They were then washed with PBS-Tween twice and treated with the secondary anti-rabbit antibody Alexa Fluor 647 (Thermo Fisher Scientific; A21244, 1:100) for 30 minutes in a darkroom at room temperature. After washing, anti-horse IgG FITC (Sigma-Aldrich; F-7759, 1:10) was added for one hour at room temperature. Thin sections were washed and slides were mounted using Hoechst (Thermo Fisher Scientific; 62249, 1:1000) and ProLong^{™} Glass Antifade Mountant (Thermo Fisher Scientific; P36980). The slides were read under a confocal microscope (Leica TSC SP8 DSL Hyvolution). Images were assembled and analyzed in 3D using Imaris Viewer software.

### Statistical analysis

Data were expressed as average ± standard error and statistically analyzed with GraphPad Prism, version 9.1 for Windows (San Diego. USA). For comparisons, the following tests were used: One Way ANOVA and multiple comparisons carried out by post-hoc Tukey HSD; Two-way ANOVA followed by post hoc Bonferroni test and Student's t test. I need to complete all tests used.

### Results

### Antigen production

SARS-CoV-2 was propagated in 6 L batches using static monolayer Vero cells in serum free medium. The virus was efficiently harvested and clarified by deep filtration with almost 100% recovery. The active virus showed good stability at 4°C for at least 24 h in this condition. Concentration by TFF using a 300 kDa membrane also showed good recovery yields (> 90%) and no virus could actually be quantified in the permeate. Ultracentrifugation was applied for virus purification in two steps. After the first step, virus was recovered in many fractions of the sucrose gradient, which were then pooled, diluted and loaded onto the second gradient. The final purified material was recovered from a narrower gradient fraction (58% to 350 sucrose). Virus inactivation was effective and safe using 15 KGy gamma irradiation, considering a sterility assurance level (SAL) applied for materials that must be in contact with humans and animals. Inactivation tests showed no cytopathic effect on Vero cell monolayers after three blind passages. Testing for viral subgenomic RNA kinetics also showed no evidence of viral replication in Vero cells by 72 h of incubation, as no statistical difference in the amount of RNA was found during the kinetic evaluation (data not shown).

### Antigen analysis

The purified and inactivated virus samples were analyzed for infection, antigenic and immunogenic characteristics. The antigen purity was verified by SDS-PAGE (Figure 10A) showing only four fragments with sizes of the three viral structural proteins (N, M, E and S; Malik, 2020). In addition, the residual host cell protein was measured by specific ELISA (234.33 ng/mL). Anti-SARS-CoV-2 protein S and anti-SARS-CoV-2 protein N specific antibodies recognized both proteins in the purified and inactivated virus by Western Blotting (Figure 10B). Furthermore, these three viral proteins (N, S and M) were identified by means of LC-MS/MS analysis of the antigen (Figure 10C). In addition, the proportion of protein content estimated based on spectral counts showed that total viral proteins represent approximately 90% of the purified and inactivated antigen sample (Figure 10C). Endotoxin measurements on purified and inactivated virus samples showed a level below assay sensitivity (<0.125 EU/mL) (data not shown).

The immunogenicity of the purified and inactivated virus sample, as well as its potential toxicity *in vivo,* were evaluated by measuring the specific antibody response elicited after immunization of mice and by toxicity parameters, respectively. Figure 11A shows that the inactivated virus is immunogenic, inducing an increase in antibody production over time of immunization, as measured by ELISA, using the virus as the antigen. The presence of neutralizing antibodies against live SARS-CoV-2 (variant B.1.1.28) was also measured. Neutralizing antibody titers, as determined by CPE-VNT, after 4 doses were 1:256 on average (Figure 11B). Furthermore, repeated inoculation of the inactivated virus, in the murine model, did not produce any toxic effects in the animals (data not shown). Thus, these results allow us to use the purified and inactivated virus for the immunization of horses.

### Production of anti-SARS-CoV-2 hyperimmune plasma

Horses were injected four times with an inactivated viral suspension of SARS-CoV-2, subcutaneously, with an interval of one week. Laboratory tests did not show relevant changes in hematological parameters throughout the immunization scheme. Biochemical analysis showed normal values, except for a slight increase in total protein and gamma globulin after the four immunization doses and before the plasmapheresis procedures.

Serum samples were collected before and during immunization and antibody titers against SARS-CoV-2 proteins were monitored by ELISA and CPE-VNT. Similarly, as observed for mice, the titration of equine plasma samples also allowed us to verify the effectiveness of the immunization scheme, reaching the highest antibody titers 28 days after the primer (Figure 12A). As expected, the horses developed much higher antibody titers to the inactivated SARS-CoV-2 than the mice, allowing three batches of hyperimmune plasma to be produced for the production of immunoglobulin F(ab') 2 fragments on days 28, 35 and 49 after the primer. Furthermore, increased levels of neutralizing antibodies were also observed during the horse immunization process (Figure 12B).

Plasma was collected when anti-SARS-CoV-2 antibody levels were considered to peak. First, the horses were examined, including assessment of weight, body condition score and assessment of intestinal motility by digestive auscultation, heart and respiratory rate by cardiorespiratory auscultation. Then, the animals were subjected to automated plasmapheresis and the veterinary team monitored the animals during the entire plasma collection, which lasted an average of 3 h, during which the animals were fed and offered water, until at least 10-12 liters were collected, or the animals presented some discomfort, due to the permanence in the stalls. All procedures were carried out in accordance with good animal handling practices. After automated plasma section, there was a slight trend towards a decrease in total serum protein and gamma globulin between the 2^{nd} and 3^{rd} plasmapheresis. Baseline values for weight and serum protein occurred at most seven days after the first two plasmapheresis procedures, except for the third plasma collection. In this case, it was decided to delay another week to allow the recovery of the serum protein level (Table 2).

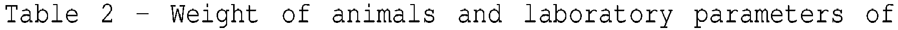

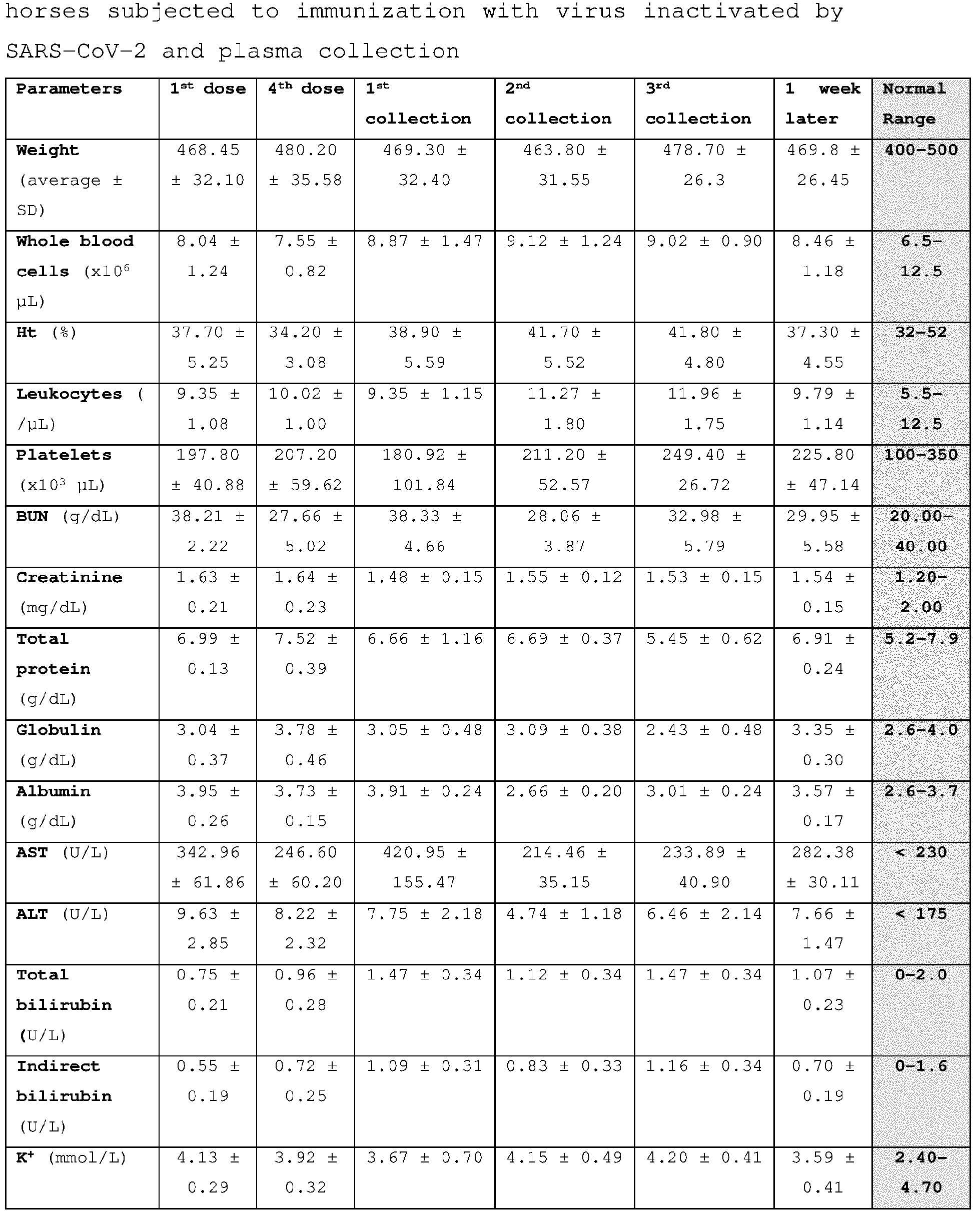

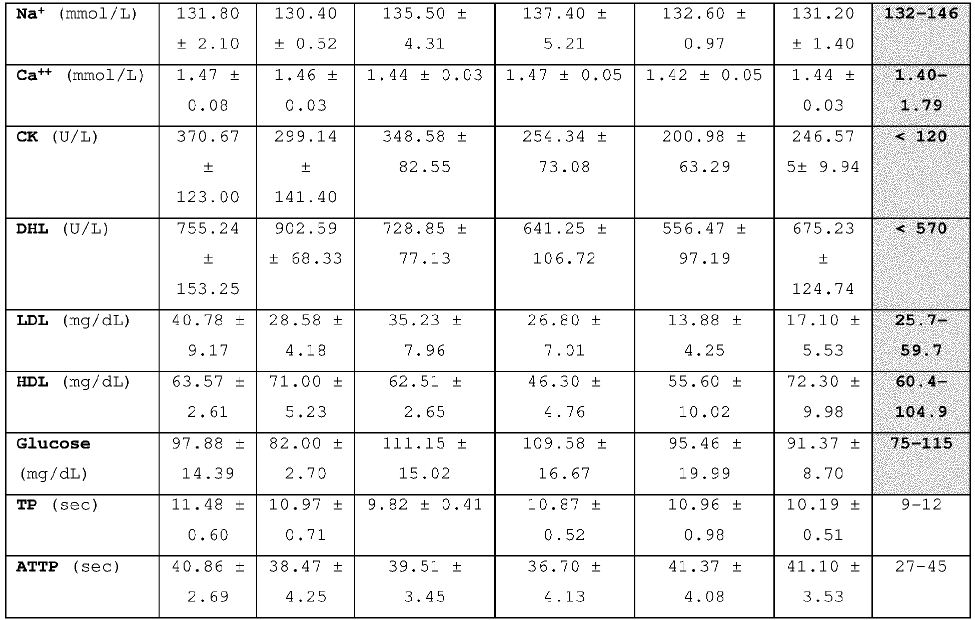

Three batches of hyperimmune plasma were obtained and the final volume of each one was composed by the sum of the plasma collected from the 10 animals at different periods of time after the immunization scheme. The plasma collection parameters are shown in Table 3. One week after automated plasmapheresis, the animals recovered to normal parameters. They were kept in a paddock with a regular supply of pasture and additional grain and water, under veterinary supervision.

**Table 3 - Collection parameters of the three lots of anti-SARS-CoV-2 hyperimmune plasma**

| Batch | Total volume collected (mL) | Collection time* (min) | Plasma volume (mL) *per* kg of animal weight* | Collection rate* (mL/min) |
|---|---|---|---|---|
| 1 | 15.082 | 257 ± 30.0 | 30.0 ± 1.8 | 54.5 ± 4.5 |
| 2 | 14.923 | 252.4 ± 27.6 | 32.5 ± 1.8 | 59.1 ± 6.4 |
| 3 | 12.710 | 230.0 ± 19.9 | 26.6 ± 4.2 | 55.1 ± 7.2 |

| | | | | |
|---|---|---|---|---|
| Anti-SARS-CoV-2 Equine Immunoglobulin Production | | | | |

Plasma batches were obtained with a total of 428.130 mL of plasma which were collected and subjected to industrial processing. Bulks resulted in three batches of purified specific fragmented anti-SARS-CoV-2 immunoglobulin, which were filled into 5.0 mL vials.

Samples from the three batches were subjected to SDS-PAGE analysis to assess the existence of proteolytic products. There were no changes in the protein profile of the three batches even after incubation for 3 h at 37°C, indicating a lack of proteolytic events from anti-SARS-CoV-2 samples (Figure 13A). In addition, it was possible to identify, through tandem mass spectrometry analysis, the presence of specific anti-Spike regions of immunoglobulins in the samples from the three batches (Figure 13B).

The samples were tested in the Quality Control of the Butantan Institute to verify the results of appearance, pH, osmolality, bottle seal, sodium chloride, phenol, ammonium sulfate, non-protein nitrogen, total solids, total protein, volume extractable, deliverable volume, purity, molecular distribution, protein aggregates, sterility and pyrogen. All results were in accordance with the Brazilian Pharmacopoeia (data not shown).

### Analysis of the potency of anti-SARS-CoV-2 hyperimmune serum

The potency analysis of anti-SARS-CoV-2 hyperimmune serum was evaluated by three different methodologies: competitive ELISA (GenScript) and CPE-VNT for detection of neutralizing antibodies and quantitative ELISA for detection of RBD domain antibodies (ROCHE) (Table 4). All three batches showed neutralizing antibodies when tested by CPE-VNT. Mean neutralizing antibody titers against the three variants currently circulating in Brazil ranged from 1120 to 2140 for variant B.1.1.28., from 100 to 200 for variant P.1. and from 1120 to 2560 for the P.2./Zeta variant (Table 4). These results agree with the results of the inhibition evaluation by competitive ELISA, in which all batches of anti-SARS-CoV-2 serum analyzed were positive (cut off = 20%) for the presence of neutralizing antibody at least up to the dilution 1/2500. Competitive ELISA was carried out under GLP conditions and the methodology was previously validated for use with equine serum. On the other hand, the quantification of total anti-Spike antibodies showed that the quantification of titers ranged from 5477.6 U / mL to 7798.3 U / mL.

**Table 4. Equine anti-SARS-CoV-2 hyperimmune serum potency**

| | **Neutralizing Antibody¹** | | **Anti-Spike²** | **Neutralizing Antibody³** | | |
|---|---|---|---|---|---|---|
| | **Dilution Factor** | **% Inhibition** | | B.1.1.28 | Gamma | Zeta |
| **SCOV200001** | 1/100 | 99.1% | 6727.5 U/mL | 1120 | 200 | 1440 |
| | 1/1000 | 72.5% | | | | |
| | 1/2500 | 43.1% | | | | |
| **SCOV200002** | 1/100 | 99.4% | 7798.3 U/mL | 2140 | 140 | 2240 |
| | 1/1000 | 76.0% | | | | |
| | 1/2500 | 43.8% | | | | |
| **SCOV200003** | 1/100 | 99.2% | 5477.6 U/mL | 1280 | 120 | 1120 |
| | 1/1000 | 50.9% | | | | |
| | 1/2500 | 28.2% | | | | |

| | | | | | | |
|---|---|---|---|---|---|---|
| ¹ 1cPass Neutralizing Antibody Detection Kit (GenScript #L00847) ² Elecsys^{®} Anti-SARS-CoV-2 S (Roche # 09289267190) ³ VNT-CPE Assay using B.1.1.28. Gamma and Zeta strains | | | | | | |

### Preclinical safety studies

The behavior of mice and rabbits was systematically observed to assess acute toxicity after 24 hours, 14 and 90 days of hyperimmune serum administration. Hippocratic analysis showed no behavioral change in mice and rabbits. There were no quantitative changes in the total weight of animals that received hyperimmune serum compared to the control and vehicle groups and, after necropsy, there were no differences in internal organ weights (data not shown). Macroscopic analysis of the internal organs after 14 and 90 days of serum administration showed no changes. No hemorrhage foci were observed in the cavities or parenchyma of the different organs, ascites or cavity fluid formation, pus formation, inflammatory exudate or obstruction in internal organs of the organs were observed.

After 14 and 90 days of serum inoculation, there was no quantitative change in the total number of erythrocytes. hematocrit or platelet number, in male and female mice (Fig. 14A and Fig. 14B). Histologically, the entire parenchyma of the internal organs is preserved.

The hematological analyzes of the rabbits showed no significant differences in the total and absolute numbers, in the number of total leukocytes, or in the number of erythrocytes and platelets (Fig. 15A and Fig. 15B). Biochemical determinations, 14 days after serum administration, did not disclose any change in acute toxicity in the groups treated with the serum compared to the control, vehicle and reference values for the species for both mice and rabbits (Fig. 16A and Fig 16B). In the histopathological analysis of the skin region and adnexa of mice from the control, vehicle and serum-treated groups, it is possible to observe the organization and preservation of the epidermis, dermis and hypodermis regions, as well as the presence of the pilum, - erector muscle, without any evidence of injury or inflammation reactions. Histopathological analyzes carried out 14 days after administration of anti-SARS-CoV-2 serum in mice treated with increased doses of anti-SARS-CoV-2 serum (33,635 U / RBD / g, 3,365 U / RBR / g and 0.3365U / RBD / g) showed no changes in the structures of the lung, hepatic and renal parenchyma. No inflammatory, degenerative or fibrotic process was detected (Figure 17).

### Hamster infection with SARS-CoV-2

Golden Syrian hamsters were used for the preclinical evaluation of the protective efficacy of anti-SARS-CoV-2 equine serum. The inventors evaluated the efficacy of treatment with equine serum carried out 2 days p.i. by assessing weight, clinical signs, viral load in the lung, trachea and nasal conchae (Fig. 5A). As expected, animals infected with SARS-CoV-2 showed progressive weight loss when compared to uninfected animals, with slight recovery on days 6 and 7 p.i. The weight changes observed in animals infected with SARS-CoV-2 that received treatment with serum were not different from animals infected with SARS-CoV-2 that received saline solution (p value > 0.05) (Figure S6A - Supplementary Material). Clinical signs of sneezing and snout rubbing were evident from day 3 p.i. in all infected animals, persisting until the last day of the experiment, and absent in uninfected animals.

Although no statistical difference was observed in respiratory tract virus isolation between treated and untreated animals (Figure 19), as measured by TCID50, the specific SARS-CoV-2 RT-qPCR showed significantly lower viral load in the lungs from animals treated with serum on day 3 p.i. when compared to animals that did not receive equine serum (Figure 18B). Viral loads were all negative for uninfected animals.

### SARS-CoV-2 pathology in the Hamster Model

On day 3 p.i., macroscopic pathological lesions consistent with hemorrhage in the lungs of animals infected with SARS-CoV-2 (G1 and G2) were observed, progressing to extensive lung involvement (often >50% of lobes) on days 3, 5 and 7 p.i. On day 7 p.i., hepatization of lung areas was common in animals infected with SARS-CoV-2. No macroscopic lesions were observed in other organs from animals infected with SARS-CoV-2, or in animals not infected with SARS-CoV-2.

Histopathological analysis of the lungs of animals infected with SARS-CoV-2 showed interstitial pneumonia, diffuse or not, with bronchoalveolar involvement, edema of the interstitium and perivascular space, leukocyte infiltration, bronchial epithelial desquamation, diffuse interstitial hemorrhage and deposition of hyaline membrane (Figure 20).

Animals infected with SARS-CoV-2, untreated, showed a more pronounced evolution of the pattern of diffuse interstitial pneumonia, bronchitis scores and edema mainly on days 3 and 5 p.i., compared to animals infected with SARS-CoV- 2, treated with serum (p value < 0.05). On days 3 and 5 p.i., SARS-CoV-2 infected untreated animals had marked neutrophilia compared to serum treated animals. The number of mononuclear cells, although not significant, increased only at 7 p.i in serum-treated animals infected with SARS-CoV-2. The control samples (uninfected animals, treated or not) showed the normal histological pattern of the tissue (Figure 21).

### Antibody detection in the COVID hamster model

Detection of horse anti-SARS-CoV-2 F(ab')2 in hamster serum samples disclosed the presence of horse immunoglobulin fragments, confirming that the intraperitoneally injected anti-SARS-CoV-2 serum , reached the bloodstream. The highest concentration was detected at 3 days p.i., in both the infected and uninfected groups, and decreased over time, similarly between the groups (Figure 22). The kinetics of infection and the protective effect of immunotherapy on lung tissue are shown in Figure 23. SARS-CoV-2 infected the bronchial alveolar epithelium and vascular endothelium and can be seen as an extracellular form in the interstitium or as an intracellular form in the leukocytes and somatic cells. The F(ab')2 fraction is distributed in the same sites as the virus, and the overlap of the F(ab')2-SARS-CoV-2 complex can be seen in detail in the 3D analysis of lung tissue from infected animals that have been treated with serum. Tissue from the saline control group was incubated with the same primary and secondary antibodies and no specific reaction was observed (data not shown).

### Discussion

The inventors show that horses immunized with purified and inactivated SARS-CoV-2 provided plasma with high antibody titers as starting material for the production of anti-SARS-CoV-2 F(ab')2 immunoglobulins, in a GMP environment.

The antigen and the final product were characterized using biochemical, immunological and biological approaches.

First, antigen inactivation was evaluated for residual virus activity after irradiation, showing no evidence of active virus demonstrating the efficacy of ionizing radiation for inactivation, as described above for other viruses and SARS-CoV -2 (Shahrudin et *al.,* 2018; Leung et *al.,* 2020; Sir Karakus et *al.,* 2020).

The production process of purified virus was also efficient in reducing the level of host cell proteins, ensuring product purity verified by MS, SDS-PAGE and quantitative ELISA.

In addition, the antigen had no contaminants and the level of endotoxins was below the accepted limit for parenteral products.

Finally, the immunological analysis of the antigen showed that mice immunized with the inactivated virus showed no sign of toxicity and generated high levels of neutralizing antibodies against SARS-CoV-2, evidencing the preservation of crucial epitopes of the S protein of the virus (Jiang & Hillye, 2020).

Anti-SARS-CoV-2 F(ab')2 immunoglobulin was evaluated using the standard physicochemical and microbiological analysis carried out for all 13 immunoglobulin products in the Butantan Institute portfolio. The new product did not show marked differences and the quality analyzes were considered adequate for the quality control of the release of lots of anti-SARS-CoV-2 immunoglobulins.

Despite showing differences in potency, the product was able to neutralize in vitro three variants of SARS-CoV-2, similarly to what is being found in several tests to establish the effectiveness of the vaccine in humans against each new variant of the virus (Salim *et al.,* 2021; Palacios *et al.,* 2021).

Golden Syrian hamsters were used for the preclinical evaluation of the protective efficacy of anti-SARS-CoV-2 equine serum. The analysis of viral load in the lung measured by qRT-PCR after 3 days of infection showed a statistical difference (p < 0.05) between animals treated and not treated with the antiserum. However, there was no statistical difference in viral load in the trachea and nasal conchae measured by both qRT-PCR and TCID50 determination between the two groups.

Histopathological analysis showed that serological treatment after 48 hours of infection impaired the development of the disease, significantly reducing edema and activation of neutrophils in the lung. These data support the potential benefits of this treatment for human patients, who often suffer from inflammation triggered by COVID-19 and neutrophil extracellular traps. It is also important to mention that the challenge studies were conducted using a single dose of antiserum, which was designed to be the lowest dose relative to the body weight of a human subject that could be injected. This may be the explanation for not detecting a sensitive reduction in viral load, as it was shown in vitro that serum lots have high neutralizing potency. In addition, Capture Elisa assays confirmed the presence of circulating equine immunoglobulins from the third to the seventh day after inoculation.

The distribution of the F(ab')2 fraction in the bronchial alveolar epithelium and endothelium, observed by immunofluorescence, showed the formation of a barrier that appeared to have a protective effect on lung tissue. Furthermore, the sequestration of F(ab')2 into lung tissue may explain its reduction in hamster serum observed in the ELISA assay.

Another important fact is that although the effects of immunotherapy on lung tissue have been described in the literature, usually with assessment of the degree of lesion and leukocyte kinetics (Frage *et al.,* 2020; Rogers et *al.,* 2020; Hassan *et al.,* 2020), in the present application, the inventors have shown the F(ab') 2-SARS-CoV-2 complex *in situ,* thus providing new information on the pharmacokinetics of immunotherapy against SARS-CoV-2.

In clinical practice, antibodies originating from horses immunized with animal venoms, bacterial toxins and rabies viruses have been used since the introduction of the first diphtheria and tetanic antitoxins in the nineteenth century. Previously, the use of equine-derived immunoglobulins has given rise to considerable problems related to early anaphylactic and non-anaphylactic reactions, and serum sickness, perhaps related to the substantial volumes of injected product. The purification steps generated much better quality products and adverse reactions decreased in frequency and severity.

Thus, antivenoms remain the cornerstone in the treatment of snakebite poisoning worldwide, while antitoxins and equine rabies immunoglobulin are still widely used, especially in low- and middle-income countries. Equine antibodies may also be given prophylactically after exposure to the organism, as in post-rabies exposure prophylaxis, in immunodeficient or immunosuppressed individuals, or when vaccines are not available.

Another possible use of equine antibody therapy, in this case for COVID-19 infection, is when patients are infected while still protected by the antibodies. In this case, the antibodies would function as a substitute vaccine, as observed for IgG antibodies against influenza, which showed protection of the lung against disease but did not protect the nose from infection in mice (Renegar *et al.,* 2004).

A phase I/II clinical trial was recently approved by the Brazilian regulatory authority and aimed to evaluate the safety, pharmacokinetics and efficacy of anti-SARS-CoV-2 F(ab') 2 equine IgG of the present application in patients in the early stage of COVID -19 at increased risk for severe cases.

In conclusion, the data presented in this application show that anti-SARS-CoV-2 F(ab')2 equine IgG can successfully neutralize the virus, is safe in animal models and is able to reduce the severity of COVID in a hamster model of SARS-CoV-2. Based on these results, the Brazilian Regulatory Agency (ANVISA) authorized the start of a clinical trial to test the efficacy of this antiserum in patients with COVID.

Therefore, the implementations of this order are in accordance with the items below:
Item 1. Process for producing an antigen corresponding to the inactivated SARS-COV-2 viruses comprising the following steps
   a) Cultivation and multiplication of the SARS-CoV-2 virus in mammalian cells,
   b) Collection,
   c) virus purification,
   d) viral stabilization, and
   e) viral inactivation
   wherein viral inactivation is by gamma irradiation and maintaining its immunogenicity.
Item 2. Process for producing an antigen, corresponding to the inactivated SARS-COV-2 viruses, according to Item 1, wherein the inactivation by gamma radiation is preferably carried out by the application of 15 kGy (kilo Grays), either for of purified virus samples as for virus samples contained in culture dishes, test tubes, microtubes, glass vials, polypropylene or other plastics.
Item 3. Process for producing an antigen, corresponding to the inactivated SARS-COV-2 viruses, according to any one of Item 1 to 2, wherein virus inactivated by gamma irradiation is immunogenic, inducing an increase in the production of antibodies over time of immunization.
Item 4. Process for producing an antigen, corresponding to the inactivated SARS-COV-2 viruses, according to Item 1, wherein in step (a) Vero cells (ATCC CCL-81.4) were seeded and cultivated for 48h prior to infection, wherein SARS-CoV-2 was inoculated at an MOI of 0.1 in 5 mL of culture medium and allowed to adsorb for one hour at 37°C, serum-free medium was added to the cultures until reaching the volume 100 ml.
Item 5. Process for producing an antigen, corresponding to the inactivated SARS-COV-2 viruses, according to Item 4, wherein the cultures were incubated at 37°C, 5% CO₂, for 48 h.
Item 6. Process for producing an antigen, corresponding to the inactivated SARS-COV-2 viruseses, according to Items 1 to 5, wherein the active SARS-CoV-2 was clarified by means of 0.22 µm filtration (Opticap XL2 Durapore , MerckMillipore) and pooled for the purification step (c).
Item 7. Process for producing an antigen, corresponding to the inactivated SARS-COV-2 viruses, according to any one of Items 1 to 6, wherein the clarified material was concentrated by tangential flow filtration (TFF) using a Minipellicon system with an Ultracell 300 kDa membrane (MerckMillipore), wherein the concentrate was subjected to two rounds of ultracentrifugation (XPN 90, BeckmanCoulter) in sucrose gradients (65% - 34%) using a Ti15 zonal rotor for 3h at 4°C and at 25 krpm, fractions containing purified virus were pooled after each ultracentrifugation procedure.
Item 8. Process for producing an antigen, corresponding to the inactivated SARS-COV-2 viruses, according to any one of Items 1 to 7, wherein the final purified material was diluted with 1X PBS to a sucrose concentration of 20%, filter sterilized (0.22 pm) and frozen at -80°C.
Item 9. Process for producing an antigen, corresponding to the inactivated SARS-COV-2 viruses, according to any one of Items 1 to 8, wherein the effectiveness of the SARS-CoV-2 inactivation process was evaluated by detecting the cytopathic effect and kinetic analysis of subgenomic viral RNA by RT-qPCR in VERO CCL 81.4. after inoculation of the SARS-CoV-2 antigen.
Item 10. Process for producing an antigen, corresponding to the inactivated SARS-COV-2 viruses, according to Item 9, wherein 0.1 mL aliquots of the inactivated virus were incubated with 1E + 05 VERO CCL - 81.4 cells for 1h at 37°C, followed by the addition of 1.4 ml of DMEM culture medium + 10% FBS, the plates were incubated for 72 hours and, in the absence of cytopathic effect, an aliquot of 0.1 ml of the supernatant was transferred to a new VERO CCL 81.4 cell culture for the second passage, the procedure was repeated at least once more.
Item 11. Process for producing an antigen, corresponding to the inactivated SARS-COV-2 viruses, according to any one of Items 9 to 10, wherein for the kinetic analysis of the subgenomic viral RNA, 1 mL of the inactivated virus was inoculated into 0.1 ml aliquots in ten wells of a 12-well plate containing VERO cells for 72 h, the supernatant from all wells was pooled and 0.1 ml aliquots were placed in a new well of a 12-well plate for the second pass in VERO cells.
Item 12. Process for producing an antigen, corresponding to the inactivated SARS-COV-2 viruses, according to any one of Items 1 to 11, wherein the purity of the inactivated virus samples was evaluated by SDS-PAGE using 4-15% CriterionTM TGXTM Precast Midi Protein gel, under nonreducing conditions (Bio-Rad), electrophoresis was performed for 1h at 100 V and the gel was stained using Pierce^{™} Silver Stain Kit (Thermo Fisher Scientific), the amount of cellular protein VERO Residual enzyme was quantified by VERO Host Cell Protein Immunoenzyme Assay, antigen purity and identity were also performed by mass spectrometry analysis.
Item 13. Process for producing an antigen, corresponding to the inactivated SARS-COV-2 viruses, according to any one of Items 1 to 12, wherein the quality of the antigen was analyzed based on the preservation of the antigen after the inactivation process by Western blotting, wherein 400 µL of the purified and inactivated antigen were subjected to SDS-PAGE, and the proteins transferred to nitrocellulose membrane for 1h at a current of 90 V - 150 mA, the membrane was blocked with the blocking solution for 3h at room temperature, under agitation, then the membrane was washed with phosphate buffer plus 0.05% Tween 20 (3 x 5 min), followed by incubation with rabbit monoclonal antibody [CR3022] for SARS-CoV-2 Spike Glycoprotein (Abcam - Ab273074) and rabbit polyclonal antibody to SARS Protein - CoV-2 N, diluted in sample diluent, for 1h, peroxidase labeled goat anti-rabbit IgG antibody (KPL) was used for marking and the membrane was developed with chemiluminescent substrate Super West Peak signal (ThermoFisher).
Item 14. Antigen, corresponding to the SARS-COV-2 viruses, inactivated, being obtained by the process as defined by any one of Items 1 to 13.
Item 15. Antigenic composition comprising the antigen, corresponding to the SARS-COV-2 viruses, inactivated as defined by Item 14 and a pharmaceutically acceptable vehicle, excipient, diluent.
Item 16. Use of the antigen, corresponding to the SARS-COV-2 viruses, inactivated as defined by Item 14 or the antigenic composition as defined by claim 15 wherein it is for producing an immunizing composition to induce the production of hyperimmune serum comprising immunoglobulin in animals.
Item 17. Use of the antigen, corresponding to the SARS-COV-2 viruses, according to Item 16, wherein the immunoglobulin is preferably equine anti-SARS-CoV-2 immunoglobulin.
Item 18. Use of the antigen, corresponding to the inactivated SARS-COV-2 viruses as defined by Item 14, wherein it is in different animal species for producing antibodies/inputs to the research and serodiagnostic kits. Item 19. Use of the antigen, corresponding to the inactivated SARS-COV-2 viruses as defined by Item 14, wherein it is for the preparation of a composition intended for immunization for the protection of the immunized individual or animal.
Item 20. Process for producing anti-SARS-CoV-2 immunoglobulin by means of the immunization of different animal species with the antigen, corresponding to the inactivated SARS-COV-2 viruses as defined by Item 14, wherein it comprises the following steps:
   a) administer the antigen corresponding to the inactivated SARS-COV-2 virus as defined by claim 14 or the antigenic composition as defined by claim 15 to an animal species;
   b) collect hyperimmune plasma from a sample obtained from the immunization of said animal species;
   c) purifying the immunoglobulins;
   d) assess serum antibody titers.
Item 21. Process producing anti-SARS-CoV-2 immunoglobulin, according to Item 20, wherein the immunization for producing anti-SARS-CoV-2 immunoglobulin is preferably in horses.
Item 22. Process for producing anti-SARS-CoV-2 immunoglobulin, according to Item 20, wherein the initial plasma pool was subjected to fractionation by precipitation with ammonium sulfate, wherein the precipitate was centrifuged and the liquid fraction was discharged, where the product was then solubilized and the pH was adjusted for enzymatic digestion by pepsin to hydrolyze the non-IgG proteins and cleave the molecule into F(ab')2 fragments, removing the Fc fragment.
Item 23. Process for producing anti-SARS-CoV-2 immunoglobulin, according to Item 20, wherein a second precipitation was carried out by adding ammonium sulfate and the pH was adjusted by adding caprylic acid and stirring, then, thermocoagulation at 56°C separated the nonspecific thermolabile proteins and the precipitate was eliminated, wherein the supernatant was submitted to diafiltration, ion exchange chromatography and concentrated by tangential ultrafiltration; phenol was added as a preservative and the chloride concentration was adjusted, where the F(ab')2 solution was then subjected to 0.22 µm filtration and kept in disposable containers.
Item 24. Process for producing anti-SARS-CoV-2 immunoglobulin, according to any one of Items 20 to 23, wherein the protein profile of the final product was analyzed using SDS-PAGE (12.5%) under conditions of reduction, where to assess the existence of any proteolytic products, the sample was incubated at 37°C for 30 min, 1 h, 2 h or 3 h and submitted to SDS-PAGE, wherein the gels were stained using Coomassie Blue dye (40% methanol, 10% acetic acid and 0.25% Coomassie blue) and destined using 40% methanol with 10% acetic acid.
Item 25. Process for producing anti-SARS-CoV-2 immunoglobulin, according to Items 20 to 24, whrein an ELISA was used to detect anti-SARS-CoV-2 IgG to evaluate obtained the serum antibody titers, ELISA plates (Costar^{®}) were coated with 100 µl of purified and inactivated SARS-CoV-2 (4.2 x 104 virus/ml) and incubated overnight at 4°C. Plates were then blocked with 5% BSA in 1X PBS for 2 h at 37°C and incubated with increasing dilutions of non-immune or experimental serum samples for 1h at room temperature, after incubation, the plates were washed with PBS-Tween 20 0.05% and incubated with anti-mouse IgG-HRPO antibodies (1:2000) or anti-horse IgG-HRPO antibodies (1:5000) in 0.1% BSA/PBS, then plates were washed and reactions performed with TMB substrate (BD OptEIA^{™}), absorbances were recorded in an ELISA reader (BioTek Elx800 spectrophotometer) at 450 nm, where the titer was established as the dilution of highest serum wherein the measured absorbance was twice as high as that determined for normal mouse serum (control group) or for pre-immune horse plasma.
Item 26. Process for producing anti-SARS-CoV-2 immunoglobulin, according to any one of Items 20 to 25, wherein a virus neutralization test based on cytopathic effect (CPE-VNT) was carried out, wherein to evaluate serum antibody titers, CPE-VNT was carried out using the wild-type B.1.1.28 variant SARS-CoV-2 (SARS-CoV-2/human/BRA/SP02/2020 (MT126808.1), wherein the final product (equine anti-SARS-CoV-2 serum) was also tested against the P.1./Gamma SARS-CoV-2 variant (IMT 87201 strain) and P.2./Zeta variant (hCoV-19 / Brazil / RS-00601/2020 - LMM- EPI_ISL_779155), wherein the assays were performed in 96-well plates seeded 24 to 28h before with 2 × 10⁴ cells / VERO cell well (ATCC CCL-81.4.), where one serial dilution of each serum (1:20 to 1:40960) in DMEM with 2.5% FBS was carried out and then 100 TCID50 of virus (v/v), virus/serum mixture was incubated at 37°C for 1h to allow virus neutralization, then, the mixture was transferred to the confluent VERO cell monolayer and incubated for 72h at 37°C, under 5% CO₂, after the incubation period, the plates were again microscopically inspected for CPE caused by SARS-CoV-2 and subsequently stained with 0.2% black naphthol blue solution and analyzed to confirm the titer.
Item 27. Process for producing anti-SARS-CoV-2 immunoglobulin, according to any one of Items 20 to 26, wherein the average titers of neutralizing antibodies against three variants range from 1120 to 2140 for variant B.1.1. 28; from 100 to 200 for variant P.1. range and from 1120 to 2560 for the P.2./Zeta variant.
Item 28. Vaccine composition comprising the anti-SARS-CoV-2 immunoglobulin obtained by the process as defined by any one of Items 20 to 27 and a pharmaceutically acceptable vehicle, excipient, diluent, adjuvant.
Item 29. Use of the vaccine composition as defined by Item 28, wherein it is for the preparation of a drug for the treatment of patients with COVID-19 infection.
Item 30. Diagnostic kit for COVID-19 comprising the antigenic composition as defined by Item 14 and a diluent and/or marker.
Item 31. A kit comprising the vaccine composition as defined by Item 28 and a diluent and/or adjuvant.
Item 32. Immunizing kit comprising the antigenic composition as defined by Item 14 and a diluent.
Item 33. Method for inducing the production of hyperimmune serum comprising the administration of an effective amount of the antigen, corresponding to SARS-COV-2 viruses, inactivated as defined by Item 14 or the antigenic composition as defined by Item 15 to animals
Item 34. Method of treating patients with COVID-19 infection comprising the administration of an effective amount of the anti-SARS-CoV-2 immunoglobulin obtained by the process as defined by any one of Items 20 to 27 or the composition vaccine as defined by Item 28 on the patient.
Item 35. Method of preventing infection by COVID-19 comprising the administration of an effective amount of the anti-SARS-CoV-2 immunoglobulin obtained by the process as defined by any one of Items 20 to 27 or of the vaccine composition as defined by Item 28 to the patient.
Item 36. COVID-19 diagnostic method comprising placing antigen, corresponding to SARS-COV-2 viruses, inactivated obtained by the process as defined by any one of Items 1 to 13 or the antigenic composition as defined by Item 14 in contact with a patient sample.

Thus, although only a few embodiments and examples of the present invention have been shown, it will be understood that various omissions, substitutions and changes in the process of producing an antigen corresponding to the inactivated SARS-CoV-2 viruses of the present invention can be made by a person skilled in the art, without departing from the spirit and scope of the present invention.

It is expressly provided that all combinations of elements that perform the same function in substantially the same way, to achieve the same results, are within the scope of the invention. Substitutions of elements from one described embodiment to another are also fully intended and contemplated.

It is also necessary to understand that the drawings are not necessarily to scale, but that they are only conceptual in nature. The intent is, therefore, to be limited, as indicated by the scope of the appended claims.

### REFERENCES

ANVISA 2013. Guide for the Conduct of Non-Clinical Studies of Toxicology and Pharmacological Safety Necessary for the Development of Drugs. http://antigo.anvisa.gov.br/documents/33836/2492465/Guia+pa ra+a+Condu%C3%A7%C3%A3o+de+Estudos+N%C3%A3o+Cl%C3%ADnicos+d e+Toxicologia+e+Seguran%C3%A7a+Farmacol%C3%B3gica+Necess%C3 %Alrios+ao+Desenvolvimento+de+Medicamentos+-+Vers%C3%A3%+2/a8cad67c-14c8-4722-bf0f-058a3a284f75.
Araujo DB, Machado RRG, Amgarten DE, Malta F de M, de Araujo GG, Monteiro CO, et al. SARS-CoV-2 isolation from the first reported patients in Brazil and establishment of a coordinated task network. Member of the Inst Oswaldo Cruz. 2020;115(12):1-8.
Burnouf T, Seghatchian J. Ebola virus convalescent blood products: Where we are now and where we may need to go. Transfus Apher Sci (2014). 51(2):120-5.
Casadevall A, Joyner MJ, Pirofski LA. A Randomized Trial of Convalescent Plasma for COVID-19 - Potentially Hopeful Signals. JAMA - J Am Med Assoc. 2020;324(5) :455-7.
Casadevall A, Pirofski LA. Antibody-mediated regulation of cellular immunity and the inflammatory response. Trends Immunol (2003). Sep;24(9):474-8.
Casadevall A, Scharff MD. Serum therapy revisited: Animal models of infection and development of passive antibody therapy. Antimicrob Agents Chemother (1994). Aug;38(8):1695-702.
Cohn CS, Estcourt L, Grossman BJ, Pagano MB, Allen ES, Bloch EM. et al. COVID-19 convalescent plasma: Interim recommendations from the AABB. Transfusion. 2021;61(4) :1313-23.
Corman, V.M.; Landt. O.; Kaiser. M.; Molenkamp. R.; Meijer. THE.; Chu. D.K.; Bleicker. T.; Brunink. S.; Schneider. J.; Schmidt. M.L.; et al. Detection of 2019 novel coronavirus (2019-nCoV) by real-time RT-PCR. Euro Survey. 2020. Jan;25(3):2000045.
Du L, He Y, Zhou Y, Liu S, Zheng BJ, Jiang S. The spike protein of SARS-CoV - A target for vaccine and therapeutic development. Nat Rev Microbiol 7. 226-236 (2009)
Fagre AC, Manhard J, Adams R, Eckley M, Zhan S, Lewis J, Rocha SM, Woods C, Kuo K, Liao W, Li L, Corper A, Challa D, Mount E, Tumanut C, Tjalkens RB , Aboellail T, Fan X, Schountz T. A Potent SARS-CoV-2 Neutralizing Human Monoclonal Antibody That Reduces Viral Burden and Disease Severity in Syrian Hamsters. Front Immunol. 2020 Dec 18;11:614256.
Focosi D, Anderson AO, Tang JW, Tuccori M. Convalescent plasma therapy for covid-19: State of the art. Clin Microbiol Rev (2020) Clin Microbiol Ver. 33(4): e00072-20.
Geramizadeh B, Marzban M. Histopathologic Findings of Coronavirus in Lung: A Mini-Review. Clin Pathol. 2020 Oct 12; 13:2632010X20951823.
Gruber AD, Osterrieder N, Bertzbach LD, Vladimirova D, Greuel S, Ihlow J, Horst D, Trimpert J, Dietert K. Standardization of Reporting Criteria for Lung Pathology in SARS-CoV-2-infected Hamsters: What Matters? Am J Respir Cell Mol Biol. 2020 Dec;63(6):856-859.
Gunn BM, Yu WH, Karim MM, Brannan JM, Herbert AS, Wec AZ, Halfmann PJ, Fusco ML, Schendel SL, Gangavarapu K, et al. A Role for Fc Function in Therapeutic Monoclonal Antibody-Mediated Protection against Ebola Virus. Cell Host Microbe (2018);24(2):221-233.e5.
Hassan AO, Case JB, Winkler ES, Thackray LB, Kafai NM, Bailey AL, McCune BT, Fox JM, Chen RE, Alsoussi WB, Turner JS, Schmitz AJ, Lei T, Shrihari S, Keeler SP, Fremont DH, Greco S, McCray PB Jr, Perlman S, Holtzman MJ, Ellebedy AH, Diamond MS. A SARS-CoV-2 Infection Model in Mice Demonstrates Protection by Neutralizing Antibodies. Cell. 2020b Aug 6;182(3):744-753.e4.
Hierholzer JC; Killington RA. Virus isolation and quantification. In Virology methods manual; Kangro H. B. M., Eds.; Academic Press Inc.: San Diego. 1996; pp. 24-32.
Hung IFN, To KKW, Lee CK, Lee KL, Chan K, Yan WW, Liu R, Watt CL, Chan WM, Lai KY. et al. Convalescent plasma treatment reduced mortality in patients with severe pandemic influenza A (H1N1) 2009 virus infection. Clin Infect Dis (2011);52(4):447-56.
JHUM (Johns Hopkins University & Medicine). COVID-19 Dashboard by the Center for Systems Science and Engineering (CSSE) at Johns Hopkins University & Medicine. 2020. https://coronavirus.jhu.edu/map.html
Jiang S, Hillyer C, Du L. Neutralizing Antibodies against SARS-CoV-2 and Other Human Coronaviruses. Trends Immunol. 2020 May;41(5):355-359.
Joyner MJ, Carter RE, Senefeld JW, Klassen SA, Mills JR, Johnson PW, et al. Convalescent Plasma Antibody Levels and the Risk of Death from Covid-19. N Engl J Med. 2021;384(11) :1015-27.
Kantha SS, A Centennial Review; the 1890 Tetanus Antitoxin Paper of von Behring and Kitasato and the Related Developments. Keio J Med (1991) Mar;40(1):35-9.
Kreye J, Reincke SM, Kornau HC, Sánchez-Sendin E, Corman VM, Liu H, Yuan M, Wu NC, Zhu X, Lee CD, Trimpert J, Höltje M, Dietert K, Stöffler L, von Wardenburg N, van Hoof S, Homeyer MA, Hoffmann J, Abdelgawad A, Gruber AD, Bertzbach LD, Vladimirova D, Li LY, Barthel PC, Skriner K, Hocke AC, Hippenstiel S, Witzenrath M, Suttorp N, Kurth F, Franke C, Endres M, Schmitz D, Jeworowski LM, Richter A, Schmidt ML, Schwarz T, Müller MA, Drosten C, Wendisch D, Sander LE, Osterrieder N, Wilson IA. Prüss H. A Therapeutic Non-self-reactive SARS-CoV-2 Antibody Protects from Lung Pathology in a COVID-19 Hamster Model. Cell. 2020;183(4) :1058-1069.e19.
León G, Herrera M, Vargas M et al. Development and characterization of two equine formulations towards SARS-CoV-2 proteins for the potential treatment of COVID-19. Sci Reports 2021; 11:9825.
León G, Herrera M, Vargas M, Arguedas M, Sánchez A, Segura Á, Gómez A, Solano G, Corrales-Aguilar E, Risner K. et al. Development and pre-clinical characterization of two therapeutic equine formulations towards SARS-CoV-2 proteins for the potential treatment of COVID-19. bioRxiv (2020) bioRxiv 2020.10.17.343863.
Leung A, Tran K, Audet J, Lavineway S, Bastien N, and Krishnan J. In Vitro Inactivation of SARS-CoV-2 Using Gamma Radiation. Applied Biosafety 2020 25:3. 157-160
Li G, Fan Y, Lai Y, Han T, Li Z, Zhou P, Pan P, Wang W, Hu D, Liu X. et al. Coronavirus infections and immune responses. J Med Virol (2020) 92 (4) :424-432
Lopardo G, Belloso WH, Nannini E et al. RBD-specific polyclonal F(ab')2 fragments of equine antibodies in patients with moderate to severe COVID-19 disease: A randomized, multicenter, double-blind, placebo-controlled, adaptive phase 2/3 clinical trial. EClinicalMedicine 2021; 34:100843.
Luke TC, Kilbane EM, Jackson JL, Hoffman SL. Meta-analysis: Convalescent blood products for Spanish influenza pneumonia: A future H5N1 treatment? Ann Intern Med. 2006 Oct 17;145(8):599-609.
Malik YA. Properties of Coronavirus and SARS-CoV-2. Malays J Pathol. 2020 Apr;42(1):3-11.
Palacios, R.; Batista, A.P.; Albuquerque, C.S.N.; Patiño, E.G.; Santos, J.D.P. Efficacy and Safety of a COVID-19 Inactivated Vaccine in Healthcare Professionals in Brazil: The PROFISCOV Study. SSRN 2021. 66.
Rajendran K, Krishnasamy N, Rangarajan J, Rathinam J, Natarajan M, Ramachandran A. Convalescent plasma transfusion for the treatment of COVID-19: Systematic review. J Med Virol (2020); 92(9):1475-1483
Renegar K, Small PA Jr, Boykins LG, Wright PF. Role of IgA versus IgG in the control of influenza viral infection in the murine respiratory tract. J Immunol 2004; 173: 1978-1986.
Roberts A, Vogel L, Guarner J, et al. Severe acute respiratory syndrome coronavirus infection of golden Syrian hamsters. J Virol. 2005;79(1):503-511.
Rogers TF, Zhao F, Huang D, Beutler N, Burns A, He WT, Limbo O, Smith C, Song G, Woehl J, Yang L, Abbott RK, Callaghan S, Garcia E, Hurtado J, Parren M, Peng L, Ramirez S, Ricketts J, Ricciardi MJ, Rawlings SA, Wu NC, Yuan M, Smith DM, Nemazee D, Teijaro JR, Voss JE, Wilson IA, Andrabi R, Briney B, Landais E, Sok D, Jardine JG, Burton DR. Isolation of potent SARS-CoV-2 neutralizing antibodies and protection from disease in a small animal model. Science. 2020 Aug 21;369(6506):956-963.
Ruifrok AC, Johnston DA. Quantification of histochemical staining by color deconvolution. Anal Quant Cytol Histol. 2001 Aug;23(4):291-9.
Salim S, Abdool Karim. New SARS-CoV-2 Variants - Clinical, Public Health, and Vaccine Implications. N Engl J Med 2021; 384:1866-1868.
Shahrudin S, Chen C, David SC, Singleton EV, Davies J, Kirkwood CD, Hirst TR, Beard M, Alsharifi M. Gamma-irradiated rotavirus: A possible whole virus inactivated vaccine. PLoS One. 2018;13(6):e0198182.
Sir Karakus G, Tastan C, Dilek Kancagi D, Yurtsever B, Tumentemur G, Demir S, Turan RD, Abanuz S, Cakirsoy D, Seyis U, Ozer S, Elibol O, Elek M, Ertop G, Arbak S, Acikel Elmas M, Hemsinlioglu C, Kocagoz AS, Hatirnaz Ng O, Akyoney S, Sahin I, Ozbek U, Telci D, Sahin F, Yalcin K, Ratip S, Ovali E. Preclinical efficacy and safety analysis of gamma-irradiated inactivated SARS-CoV-2 vaccine candidates. Sci Rep. 2021 Mar 11;11(1):5804.
Soo YOY, Cheng Y, Wong R, Hui DS, Lee CK, Tsang KKS, Ng MHL, Chan P, Cheng G, Sung JJY. Retrospective comparison of convalescent plasma with continuing high-dose methylprednisolone treatment in SARS patients. Clin Microbiol Infect (2004). 10(7): 676-678.
The RECOVERY Collaborative Group. Convalescent plasma in patients admitted to hospital with COVID-19 (RECOVERY): a randomised, controlled, open-label, platform trial. medRxiv [Internet]. 2021;2021.02.11.21249258.
Van Erp EA, Luytjes W, Ferwerda G, Van Kasteren PB. Fc-mediated antibody effector functions during respiratory syncytial virus infection and disease. Front Immunol (2019) 10:548.
Winau F, Winau R. Emil von Behring and serum therapy. Microbes Infect (2020). Feb;4(2):185-8.
Wiśniewski JR, Zougman A, Nagaraj N, Mann M. Universal sample preparation method for proteome analysis. Nat Methods. 2009; 6:359-362.
Wolfel, R., Corman, V.M., Guggemos. W. et al. Virological assessment of hospitalized patients with COVID-2019. Nature 581. 465-469 (2020).
Zhou B, Zhong N, Guan Y. Treatment with Convalescent Plasma for Influenza A (H5N1) Infection. N Engl J Med (2007) Oct 4;357(14):1450-1.

## Claims

1. Process for producing an antigen corresponding to the inactivated SARS-COV-2 viruses comprising the following steps
a) Cultivation and multiplication of the SARS-CoV-2 virus in mammalian cells,
b) Collection,
c) virus purification,
d) viral stabilization, and
e) viral inactivation
**characterized in that** the viral inactivation is by gamma irradiation and maintaining its immunogenicity.

2. Process for producing an antigen, corresponding to the inactivated SARS-CoV-2 viruses, according to claim 1, **characterized in that** the inactivation by gamma radiation is preferably carried out by the application of 15 kGy (kilo Grays), either for purified virus samples as for virus samples contained in culture dishes, test tubes, microtubes, glass vials, polypropylene or other plastics.

3. Process for producing an antigen, corresponding to the inactivated SARS-CoV-2 viruses, according to any one of claims 1 to 2, **characterized in that** the virus inactivated by gamma irradiation is immunogenic, inducing an increase in the production of antibodies over time of immunization.

4. Process for producing an antigen, corresponding to the inactivated SARS-COV-2 viruses, according to claim 1, **characterized in that** in step (a) Vero cells (ATCC CCL-81.4) were seeded and cultivated for 48h prior to infection, wherein SARS-CoV-2 was inoculated at an MOI of 0.1 in 5 mL of culture medium and allowed to adsorb for one hour at 37°C, serum-free medium was added to the cultures until reaching the volume 100 ml.

5. Process for producing an antigen, corresponding to the inactivated SARS-COV-2 viruses, according to claim 4, **characterized in that** the cultures were incubated at 37°C, 5% CO₂, for 48 h.

6. Process for producing an antigen, corresponding to the inactivated SARS-COV-2 viruses, according to any one of claims 1 to 5, **characterized in that** the active SARS-CoV-2 was clarified by means of 0.22 µm filtration (Opticap XL2 Durapore, MerckMillipore) and pooled for the purification step (c).

7. Process for producing an antigen, corresponding to the inactivated SARS-COV-2 viruses, according to any one of claims 1 to 6, **characterized in that** the clarified material was concentrated by tangential flow filtration (TFF) using a Minipellicon system with an Ultracell 300 kDa membrane (MerckMillipore), wherein the concentrate was subjected to two rounds of ultracentrifugation (XPN 90, BeckmanCoulter) in sucrose gradients (65% - 34%) using a Ti15 zonal rotor for 3h at 4°C and at 25 krpm, fractions containing purified virus were pooled after each ultracentrifugation procedure.

8. Process for producing an antigen, corresponding to the inactivated SARS-COV-2 viruses, according to any one of claims 1 to 7, **characterized in that** the final purified material was diluted with 1X PBS to a sucrose concentration of 20%, filter sterilized (0.22 pm) and frozen at -80°C.

9. Process for producing an antigen, corresponding to the inactivated SARS-COV-2 viruses, according to any one of claims 1 to 8, **characterized in that** the effectiveness of the SARS-CoV-2 inactivation process was evaluated by detecting the cytopathic effect and kinetic analysis of subgenomic viral RNA by RT-qPCR in VERO CCL 81.4. after inoculation of the SARS-CoV-2 antigen.

10. Process for producing an antigen, corresponding to the inactivated SARS-COV-2 viruses, according to claim 9, **characterized in that** 0.1 mL aliquots of the inactivated virus were incubated with 1E + 05 VERO CCL - 81.4 cells for 1h at 37°C, followed by the addition of 1.4 ml of DMEM culture medium + 10% FBS, the plates were incubated for 72 hours and, in the absence of cytopathic effect, an aliquot of 0.1 ml of the supernatant was transferred to a new VERO CCL 81.4 cell culture for the second passage, the procedure was repeated at least once more.

11. Process for producing an antigen, corresponding to the inactivated SARS-COV-2 viruses, according to any one of claims 9 to 10, **characterized in that** for the kinetic analysis of the subgenomic viral RNA, 1 mL of the inactivated virus was inoculated into 0.1 ml aliquots in ten wells of a 12-well plate containing VERO cells for 72 h, the supernatant from all wells was pooled and 0.1 ml aliquots were placed in a new well of a 12-well plate for the second pass in VERO cells.

12. Process for producing an antigen, corresponding to the inactivated SARS-COV-2 viruses, according to any one of claims 1 to 11, **characterized in that** the purity of the inactivated virus samples was evaluated by SDS-PAGE using 4-15% CriterionTM TGXTM Precast Midi Protein gel, under nonreducing conditions (Bio-Rad), electrophoresis was performed for 1h at 100 V and the gel was stained using Pierce^{™} Silver Stain Kit (Thermo Fisher Scientific), the amount of cellular protein VERO Residual enzyme was quantified by VERO Host Cell Protein Immunoenzyme Assay, antigen purity and identity were also performed by mass spectrometry analysis.

13. Process for producing an antigen, corresponding to the inactivated SARS-COV-2 viruses, according to any one of claims 1 to 12, **characterized in that** the quality of the antigen was analyzed based on the preservation of the antigen after the inactivation process by Western blotting, wherein 400 µL of the purified and inactivated antigen were subjected to SDS-PAGE, and the proteins transferred to nitrocellulose membrane for 1h at a current of 90 V - 150 mA, the membrane was blocked with the blocking solution for 3h at room temperature, under agitation, then the membrane was washed with phosphate buffer plus 0.05% Tween 20 (3 x 5 min), followed by incubation with rabbit monoclonal antibody [CR3022] for SARS-CoV-2 Spike Glycoprotein (Abcam - Ab273074) and rabbit polyclonal antibody to SARS Protein - CoV-2 N, diluted in sample diluent, for 1h, peroxidase labeled goat anti-rabbit IgG antibody (KPL) was used for marking and the membrane was developed with chemiluminescent substrate Super West Peak signal (ThermoFisher).

14. Antigen, corresponding to the SARS-COV-2 viruses, inactivated **characterized by** being obtained by the process as defined by any one of claims 1 to 13.

15. Antigenic composition **characterized by** comprising the antigen, corresponding to the SARS-COV-2 viruses, inactivated as defined by claim 14 and a pharmaceutically acceptable vehicle, excipient, diluent.

16. Use of the antigen, corresponding to the SARS-COV-2 viruses, inactivated as defined by claim 14 or the antigenic composition as defined by claim 15 **characterized in that** it is for producing an immunizing composition to induce the production of hyperimmune serum comprising immunoglobulin in animals.

17. Use of the antigen, corresponding to the SARS-COV-2 viruses, according to claim 16, **characterized in that** the immunoglobulin is preferably equine anti-SARS-CoV-2 immunoglobulin.

18. Use of the antigen, corresponding to the inactivated SARS-COV-2 viruses as defined by claim 14, **characterized in that** it is in different animal species for producing antibodies/inputs to the research and serodiagnostic kits.

19. Use of the antigen, corresponding to the inactivated SARS-COV-2 viruses as defined by claim 14, **characterized in that** it is for the preparation of a composition intended for immunization for the protection of the immunized individual or animal.

20. Process for producing anti-SARS-CoV-2 immunoglobulin by means of the immunization of different animal species with the antigen, corresponding to the inactivated SARS-COV-2 viruses as defined by claim 14, **characterized in that** it comprises the following steps:
a) administer the antigen corresponding to the inactivated SARS-COV-2 virus as defined by claim 14 or the antigenic composition as defined by claim 15 to an animal species;
b) collect hyperimmune plasma from a sample obtained from the immunization of said animal species;
c) purify the immunoglobulins;
d) assess serum antibody titers.

21. Process for producing anti-SARS-CoV-2 immunoglobulin, according to claim 20, **characterized in that** the immunization for producing anti-SARS-CoV-2 immunoglobulin is preferably in horses.

22. Process for producing anti-SARS-CoV-2 immunoglobulin, according to claim 20, **characterized in that** the initial plasma pool was subjected to fractionation by precipitation with ammonium sulfate, wherein the precipitate was centrifuged and the liquid fraction was discharged, where the product was then solubilized and the pH was adjusted for enzymatic digestion by pepsin to hydrolyze the non-IgG proteins and cleave the molecule into F(ab')2 fragments, removing the Fc fragment.

23. Process for producing anti-SARS-CoV-2 immunoglobulin, according to claim 20, **characterized in that** a second precipitation was carried out by adding ammonium sulfate and the pH was adjusted by adding caprylic acid and stirring, then, thermocoagulation at 56°C separated the nonspecific thermolabile proteins and the precipitate was eliminated, wherein the supernatant was submitted to diafiltration, ion exchange chromatography and concentrated by tangential ultrafiltration; phenol was added as a preservative and the chloride concentration was adjusted, where the F(ab')2 solution was then subjected to 0.22 µm filtration and kept in disposable containers.

24. Process for producing anti-SARS-CoV-2 immunoglobulin, according to any one of claims 20 to 23, **characterized in that** the protein profile of the final product was analyzed using SDS-PAGE (12.5%) under conditions of reduction, where to assess the existence of any proteolytic products, the sample was incubated at 37°C for 30 min, 1 h, 2 h or 3 h and submitted to SDS-PAGE, wherein the gels were stained using Coomassie Blue dye (40% methanol, 10% acetic acid and 0.25% Coomassie blue) and destined using 40% methanol with 10% acetic acid.

25. Process for producing anti-SARS-CoV-2 immunoglobulin, according to claims 20 to 24, **characterized in that** an ELISA was used to detect anti-SARS-CoV-2 IgG to evaluate obtained the serum antibody titers, ELISA plates (Costar^{®}) were coated with 100 µl of purified and inactivated SARS-CoV-2 (4.2 x 104 virus/ml) and incubated overnight at 4°C. Plates were then blocked with 5% BSA in 1X PBS for 2 h at 37°C and incubated with increasing dilutions of non-immune or experimental serum samples for 1h at room temperature, after incubation, the plates were washed with PBS-Tween 20 0.05% and incubated with anti-mouse IgG-HRPO antibodies (1:2000) or anti-horse IgG-HRPO antibodies (1:5000) in 0.1% BSA/PBS, then plates were washed and reactions performed with TMB substrate (BD OptEIA^{™}), absorbances were recorded in an ELISA reader (BioTek Elx800 spectrophotometer) at 450 nm, where the titer was established as the dilution of highest serum wherein the measured absorbance was twice as high as that determined for normal mouse serum (control group) or for pre-immune horse plasma.

26. Process for producing anti-SARS-CoV-2 immunoglobulin, according to any one of claims 20 to 25, **characterized in that** a virus neutralization test based on cytopathic effect (CPE-VNT) was carried out, wherein to evaluate serum antibody titers, CPE-VNT was carried out using the wild-type B.1.1.28 variant SARS-CoV-2 (SARS-CoV-2/human/BRA/SP02/2020 (MT126808.1), wherein the final product (equine anti-SARS-CoV-2 serum) was also tested against the P.1./Gamma SARS-CoV-2 variant (IMT 87201 strain) and P.2./Zeta variant (hCoV-19 / Brazil / RS-00601/2020 - LMM- EPI_ISL_779155), wherein the assays were performed in 96-well plates seeded 24 to 28h before with 2 x 10⁴ cells / VERO cell well (ATCC CCL-81.4.), where one serial dilution of each serum (1:20 to 1:40960) in DMEM with 2.5% FBS was carried out and then 100 TCID50 of virus (v/v), virus/serum mixture was incubated at 37°C for 1h to allow virus neutralization, then, the mixture was transferred to the confluent VERO cell monolayer and incubated for 72h at 37°C, under 5% CO₂, after the incubation period, the plates were again microscopically inspected for CPE caused by SARS-CoV-2 and subsequently stained with 0.2% black naphthol blue solution and analyzed to confirm the titer.

27. Process for producing anti-SARS-CoV-2 immunoglobulin, according to any one of claims 20 to 26, **characterized in that** the average titers of neutralizing antibodies against three variants range from 1120 to 2140 for variant B.1.1. 28; from 100 to 200 for variant P.1. range and from 1120 to 2560 for the P.2./Zeta variant.

28. Vaccine composition **characterized in that** it comprises the anti-SARS-CoV-2 immunoglobulin obtained by the process as defined by any one of claims 20 to 27 and a pharmaceutically acceptable vehicle, excipient, diluent, adjuvant.

29. Use of the vaccine composition as defined by claim 28, **characterized in that** it is for the preparation of a drug for the treatment of patients with COVID-19 infection.

30. Diagnostic kit for COVID-19 **characterized by** comprising the antigenic composition as defined by claim 14 and a diluent and/or marker.

31. Kit **characterized by** comprising the vaccine composition as defined by claim 28 and a diluent and/or adjuvant.

32. Immunizing kit **characterized by** comprising the antigenic composition as defined by claim 14 and a diluent.

33. Method to induce the production of hyperimmune serum **characterized by** comprising the administration of an effective amount of the antigen, corresponding to the SARS-COV-2 viruses, inactivated as defined by claim 14 or the antigenic composition as defined by claim 15 in animals.

34. Method of treating patients with COVID-19 infection **characterized by** comprising the administration of an effective amount of anti-SARS-CoV-2 immunoglobulin obtained by the process as defined by any one of claims 20 to 27 or the composition vaccine as defined by claim 28 in the patient.

35. Method of preventing infection by COVID-19 **characterized by** comprising the administration of an effective amount of the anti-SARS-CoV-2 immunoglobulin obtained by the process as defined by any one of claims 20 to 27 or of the vaccine composition as defined by claim 28 to the patient.

36. COVID-19 diagnostic method **characterized by** comprising placing antigen, corresponding to the SARS-COV-2 viruses, inactivated, obtained by the process as defined by any one of claims 1 to 13 or the antigenic composition as defined by claim 14 in contact with a patient sample.
